# EUROPEAN PATENT SPECIFICATION

(11) **EP 2 262 862 B1**
(45) Date of publication and mention of the grant of the patent: **28.03.2012**
(21) Application number: 08784429.6
(22) Date of filing: 28.08.2008
(51) Int. Cl.: C09B 61/00, A23L 1/275, C12M 1/06, C12M 1/16

(54) **PRODUCTION OF MONASCUS-LIKE AZAPHILONE PIGMENT**
HERSTELLUNG VON MONASCUSÄHNLICHEM AZAPHILONPIGMENT
PRODUCTION DE PIGMENT D'AZAPHILONE MONASCUS

(30) Priority: 28.08.2007 US 966440 P
(43) Date of publication of application: 22.12.2010
(73) Proprietor: DTU, Technical University Of Denmark, 2800 Lyngby (DK)
(72) Inventor: MAPARI, Sameer, A., S., DK-2880 Bagsvaerd (DK); MEYER, Anne, S., DK-2900 Hellerup (DK); FRISVAD, Jens, C., DK-2800 Kgs. Lyngby (DK); THRANE, Ulf, DK-3200 Helsinge (DK)
(74) Representative: Nilausen, Kim
(86) International application number: PCT/DK2008/000306
(87) International publication number: WO 2009/026923

(56) References cited:
- EP-A- 1 884 562
- WO-A-00/37665
- WO-A-01/07563
- WO-A-02/11563
- WO-A-93/16962
- WO-A-2005/016826
- WO-A-2006/028263
- DE-B- 1 084 437
- FR-A- 1 053 367
- GB-A- 1 238 401
- GB-A- 2 348 876
- US-A- 2 094 004
- US-A- 3 017 951
- US-A- 4 929 452
- US-A- 5 075 234
- US-B1- 6 340 586
- US-B1- 6 979 308
- DATABASE WPI Week 199201 Thomson Scientific, London, GB; AN 1992-002457 XP002530877 & JP 03 254686 A (JAPAN STEEL WORKS LTD) 13 November 1991 (1991-11-13)
- DATABASE WPI Week 197516 Thomson Scientific, London, GB; AN 1975-26499W XP002530878 & JP 49 093587 A (AMANO PHARM KK) 5 September 1974 (1974-09-05)

## Description

### Technical field of the invention

The present invention relates to the field of biotechnological production of polyketide based colorants from filamentous fungi, in particular a method for preparing a *Monascus*-like pigment composition from a nontoxigenic and non-pathogenic fungal source. The present invention further relates to use of the *Monascus-like* pigment composition as a colouring agent in food items and/or non-food items, and in cosmetic compositions.

### Background of the invention

Currently, the European Union has authorized approximately 43 colorants as food additives, while approximately 30 colour additives are approved for food use in the US, and several of the listed colour additives are derived from natural sources typically by physical and/or chemical extraction.

The existing authorized natural food colorants are mostly of plant origin and have numerous drawbacks such as chemical instability and low water solubility. For instance betanins, carotenoids, and chlorophyll pigments contain labile hydrogen, and are therefore easily decolorised by oxidation, and hence sensitive to light, heat and oxygen. These features limit the robustness of these colour additives during the processing, storage, and display of the foods to which they have been added. Naturally derived colorants are usually extracted from sources such as fruit skins, seeds, or roots, which are often not available throughout the year. This means that the colour manufacturers are dependent on the availability and external supply of raw materials for the colour extraction.

Fungi provide an alternative source of natural colours, since they can be produced all year round. Considering the extraordinary diversity of fungal pigments and the potential to produce them in higher yields *e.g.* by metabolic engineering, fungi could be a promising source of colorants with improved functionality over existing natural food colorants with similar or additional colour hues in the red and yellow spectra.

Fungal pigments have mostly been studied from a taxonomic perspective and earlier work on their application for food use has focused on carotenoids.

Many fungi have been reported to produce polyketide pigments but only a few of those have been explored as possible food colorants. Some of these include yellow-red compounds produced by *Penicillium herquei,* blue-green and dark green pigments of *Penicillium atrovenetum.*

*Monascus* is a genus of mold whose members produce red-yellow polyketide pigments comprising six main polyketide based azaphilone chromophores: monascorubrin, monascin, monascorubramine, rubropunctatin, rubropunctamine, and ankaflavin. Among the 24 known species of this genus, the red-pigmented *Monascus purpureus* is among the most important because of its traditional use as a natural colorant in the production of certain fermented foods in East Asia, particularly China and Japan. For example, *Monascus* species viz. *Monascus ruber* and *Monascus purpureus* have been used in red alcohol beverages, red rice and tofu. However, one drawback of pigments produced by *Monascus* species is the (risk of) co-production of citrinin, a toxic metabolite of *Monascus purpureus.* The co-production of citrinin has meant that the use of *Monascus* species as producers of natural colorants for food use is not permitted in the European Union and in the US. Another drawback of the pigments produced by *Monascus* species is that they are not particularly light-stable. Therefore, some effort has been made to improve the light stability of *Monascus* pigments, *e.g.* by cultivating the *Monascus* strain in the presence of amino acids as described by Jung et al. (J. Agric. Food Chem. 2005, 53, 7108-7114).

Mapari et al. (Curr. Opin. Biotechnol. 2005, 16, 231-238) explores fungal biodiversity for the production of water-soluble pigments as potential natural food colorants. Mapari et al. (J. Agric. Food Chem. 2006, 54(19), 7027-7035) compares the colour characteristics of 18 different strains of ascomycetous fungi with 11 commercially available colourants and shows that there exists a pigment-producing genera of ascomycetous fungi other than *Monascus* that produce similar colours.

WO 2006/028263 Al disclose an erasable ink containing at least one kind of a violet dye that can be produced by a *Penicillium fungus,* and a yellow dye that can be produced by a *Monascus fungus.* Those dyes, which can be produced by specific microorganisms, may be mixtures containing other components without particular limitation as long as they can be used for ink.

JP 3254686 A (AN=1992-002457) disclose, according to Thomson Scientific database WPI, extraction of pigment from cell body of pigment-producing microorganism - using ultrasonic waves, with pigment being used in foods, cosmetics, etc.

US 4929452 disclose a method for rapidly fermenting alcoholic beverages.

US 6 979 308 disclose a bioreactor design and process for engineering tissue from cells.

Jun Ogihara et al. (2000) Journal of bioscience and Bioengineering, 90(5), pp. 549-554 disclose the production and structural analysis of PP-V, a homologue of monascorubramine, produced by a new isolate of *Penicillium sp.*

### Summary of the invention

The inventors have found that *Monascus*-like pigment compositions are produced in some *Penicillium* strains, notably:
*P. neopurpurogenum* (IBT 11181, CBS 238.95, CBS 123796)
*P. neopurpurogenum* (IMI 90178, IST 4428, IBT 3645, CBS 113154)
*P. neopurpurogenum* ((NRRL 1136, IBT 3458, CBS 113153)
*P. neopurpurogenum* (CBS 364.48, IBT 4529)
*P. neopurpurogenum* (NRRL 1748, IST 3933)
*P. neopurpurogenum* (FRR 75, IBT 4454)
*P. aculeatum* (FRR 2129, IBT 14259, IBT 4185)
*P. pseudoaculeatum* (IMI 133243, IBT 14129)
*P. rubroaculeatum* (FRR 2005, IBT 14256)
*P*. *rubroaculeatum* (FRR 1664, IBT 14254)
*P*. *pinophilum* (IMI 114993, IBT 3757)
*P. quasipinophilum* (ATCC 9644, IBT 13104)
*P. neominioluteum* (CCRC 32646, IBT 18368)
*P. punicae* (RMF 81.01, IBT 23082)
*P. synnemafuniculosum* (NRRL 2119, IBT 3954)
*P. amestolkiae* (IMI 147406, IBT 21723)
*P.* monascurn (WSF 3955, IBT 14065, CBS 123797)
¹ Presently classified as *P. purpurogenum;* ² Presently classified as *P. aculeatum;* ³ Presently classified as *P. pinophilum.;* ⁴ Presently classified as *P. minioluteum.;* ⁵ new nomenclature should be given.

The inventors have further found that one advantage of these strains, is that they are non-pathogenic, and essentially non-toxigenic, *i.e.* do not produce citrinin or other known mycotoxins.

A further advantage is that the *Monascus-like* pigment compositions produced by these Penicillium strains have an increased light-stability compared to *Monascus* pigments.

Thus, in one embodiment the present invention provides a method for producing a *Monascus*-like pigment composition from these non-pathogenic and non-toxigenic strains mentioned above, comprising the following: providing an inoculum, inoculating a growth medium with the inoculum, cultivating the inoculated growth medium, harvesting a biomass product, and isolating the *Monascus*-like pigment composition from the biomass or the growth medium.

Another advantage is that the *Monascus*-like pigment compositions can be produced in the food and/or non-food itself, such as in the production of fermented beverages, cheeses, etc.

Accordingly, in another embodiment a method for colouring a food product and/or non-food product employing a *Monascus*-like pigment composition using at least one of the above-mentioned strains is provided.

The *Monascus-like* pigment compositions can be used in food products and/or non-food products, and in another embodiment an isolated *Monascus-*like pigment composition is used as a colouring agent for a food product and/or non-food product.

In another embodiment a cosmetic composition comprising a *Monascus*-like pigment composition is provided.

The Monascus-like pigments of the present invention can be produced on an industrial scale in liquid culture, taking advantage of the fact that some strains of *Penicillium* are capable of secreting Monascus-like pigments into the extracellular phase, from which the pigments may be recovered. Thus, a further embodiment provides a method for producing a *Monascus*-like pigment composition comprising: providing a liquid-phase growth medium and a solid support; producing a cultivation composition comprising combining the growth medium and the solid support, wherein at least part of the solid support is submerged in the growth medium; inoculating the solid support or the cultivation composition with an inoculum of *Penicillium* spores; cultivating the inoculated cultivation composition; separating the liquid-phase comprising the *Monascus*-like pigment composition from the product of (d).
Optionally, the solid support is retained within a semi-permeable container.

The invention further provides a bioreactor adapted for the large-scale production of a *Monascus*-like pigment composition, comprising a closable container for receiving a liquid growth medium, wherein the container is provided with a means for mixing; an inlet and outlet port for aeration of the container; an inlet port and outlet port for introduction and removal of biological material; characterised in that the bioreactor is provided with a solid support retained within the container, said solid support being capable of at least partial submersion in the liquid growth medium when present into the container. Optionally, the solid support is retained within a semi-permeable container.

### Brief description of the drawings

**Figure 1****:** Schematic illustration of intelligent screening and identification of Monascorubramine from *Penicillium neopurpurogenum* strain.
**Figure 2****:** UV chromatogram at 300-700 nm, and UV spectrum of citrinin (bottom), Total ion chromatogram (top), and high-resolution mass spectrum of citrinin present in a pigment extract of a *Monascus* strain.
**Figure 3****:** UV-vis chromatogram at 400-700 nm, and UV-vis spectrum of monascorubrin (bottom), Total ion chromatogram (top), and high resolution mass spectrum of monascorubrin present in a pigment extract of *Penicillium quasipinophilum* (ATCC 9644, IBT 13104) on YES medium.
**Figure 4****:** Light stability of some representative *Penicillium* spp. pigment extracts compared to the pigment extract of *Monascus ruber* (IBT 7904) and a commercial product: *Monascus* Red (Riken Vitamin Co. Ltd., Japan).
**Figure 5****:** Structures of exemplary *Monascus* and Monascus-like polyketide based azaphilone pigments.
**Figure 6A****:** Schematic presentation of a production process using solid support for fungal mycelia by confinement technique.
**Figure 6B****:** An illustration of a microfiltration technique employed for the concentration of pigments.
**Figure 7****:** Light Expanded Clay Aggregates (LECA) used as solid support for the fungal mycelia.
**Figure 8****:** Flasks comprising the fungus *Penicillium purpurogenum* IBT 4529, cultivated by a confinement technique as in Example 2, showing pigment production.
**Figure 9A****-C:** A bioreactor adapted for pigment production by a fungal microorganism.
**Figure 9D****:** Scale up of pigment production by a fungal microorganism in a bioreactor maintained in a water bath at 25 °C.
**Figure 10A****-B:** Calibration curve of the concentration of commercially available *Monascus* red pigment and carminic acid.
**Figure 11****:** Effect of carbon and nitrogen sources in the two liquid media (Example 8) on pigment production by *Penicillium purpurogenum* IBT11181
**Figure 12****:** Media formulation for tailor made production of red-orange pigments by *Penicillium purpurogenum* IBT11181.
**Figure 13****:** Total ion chromatogram (A), and UV-vis chromatogram at 400-700 nm (B), high-resolution mass spectrum (A1) and UV-vis spectrum of N-glutarylmonascorubramine (B1), high-resolution mass spectrum (A2) and UV-vis spectrum of N-glutarylrubropunctamine (B2) present in the partially purified pigment extract of *Penicillium purpurogenum* IBT 11181 in control medium as in Example 9.
**Figure 14****:** Total ion chromatogram (C), and UV-vis chromatogram at 400-700 nm (D), high resolution mass spectrum (C1) and UV-vis spectrum of N-glutarylmonascorubramine (D1) present in the partially purified pigment extract of *Penicillium purpurogenum* IBT 3645 in N11 medium as in Example 10.

### Detailed description of the invention

Fungi provide an alternative source of natural colours, since they can be produced all year round. Considering the extraordinary diversity of fungal pigments and the potential to produce them in higher yields *e.g.* by metabolic engineering, fungi could be a promising source of colorants with improved functionality over existing natural food colorants with similar or additional colour hues in the red and yellow spectra.

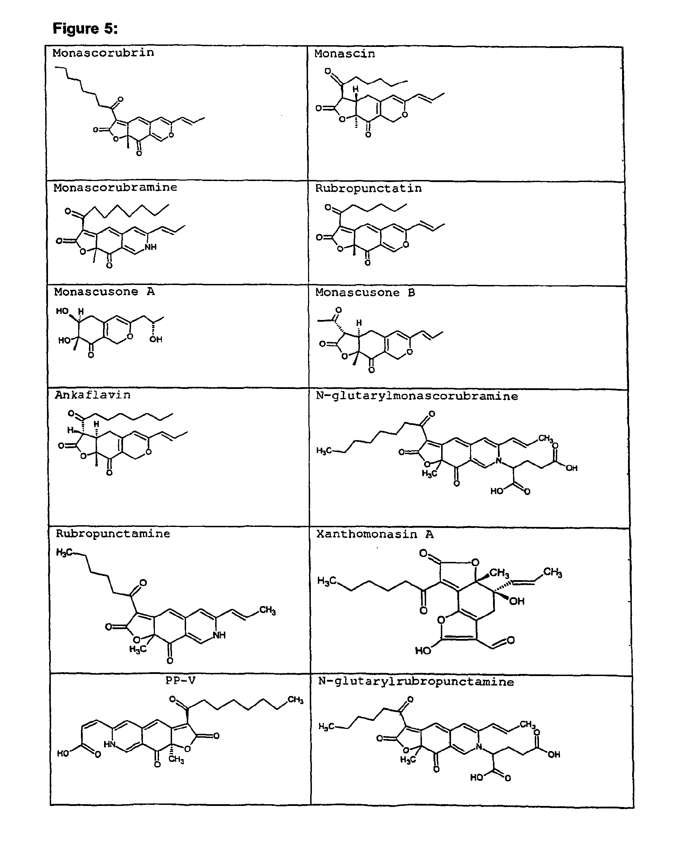

The present invention provides a novel source of polyketide-based, azaphilone pigment suitable for use in both the food and non-food industries, and methods for its production. These pigments are classified as *Monascus-*like pigments, since they comprise azaphilones and derivatives thereof, several of which are common to *Monascus*-derived pigments (Figure 5).

### 1. Screening for Monascus-like pigment producers

The identification of this novel source of *Monascus*-like pigments was based on screening of fungal populations for the ability to produce *Monascus*-like pigments suitable for the food and non-food industries. The screening process focused on ascomycetous fungi, since fungi from this family comprise members that are suitable for growth under laboratory and/or industrial scale.

Within the Ascomycetes, members of the genus *Penicillium* was selected for screening, since this genus is distantly related to the *Monascus* genus, and therefore a potential candidate to a source of *Monascus*-like pigments.

The genus Penicillium has over 1000 members, and many are yet to be described. Thus, a screening procedure is needed. Such a screening procedure is illustrated in the following:

### I.I Overview of a screening procedure:

1. Select fungi, media, and cultivation conditions based on chemotaxonomy.
2. Grow (culture) selected fungi on selected media and incubate for 7 days at 25 °C in the dark.
3. Extract pigment by micro-scale extraction method from fungal cultures.
4. Perform chromatographic analysis of fungal pigment extracts by HPLC-DAD-MS.
5. Identify metabolites on the basis of their similarity to UVNIS and mass spectra of known metabolites found by searching in both in-house and public databases.

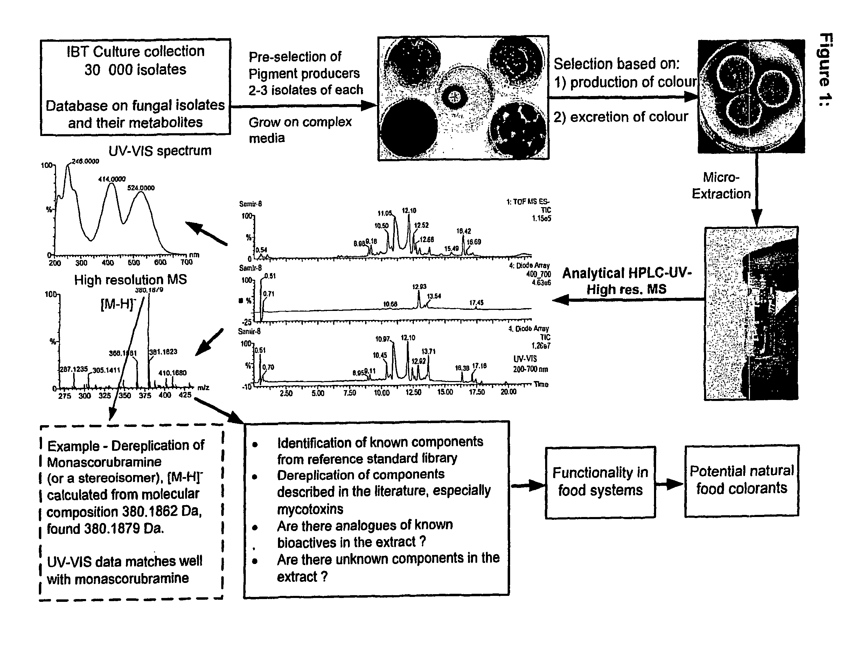

Dereplication can be used to determine if the strain produces mycotoxins, and to determine if the pigment produced is *Monascus*-like, i.e. contains polyketide based azaphilone colorant(s). Figure 1 schematically illustrates a procedure for the screening and identification of fungal pigments as potential natural food colorants. Pigment producers are initially preselected from a culture collection such as the IBT fungal culture collection at the Center of Microbial Biotechnology, DTU, Kgs. Lyngby, Denmark. Selection is made on the basis of colour production or excretion, and microextraction is used to prepare a sample for analysis. Analytical HPLC analysis is employed to identify coloured compounds. In figure 1, the central panel depicts three HPLC chromatograms (from top): total ion chromatogram (*m*/*z* 100-900) from negative electrospray mass spectrometry, UVNIS chromatogram of 400-700 nm showing coloured compounds, and UVNIS chromatogram from 200-700 nm also showing non-coloured compounds. Identification of a pigment (*e.g.* monascorubramine) is shown on the left-hand side, where the mass of the deprotonated molecular ion [M-H]⁻ is used to calculate the molecular composition of C₂₃H₂₇NO₄. The information of the molecular composition is combined with the UVNIS spectrum. Comparison with the UVNIS spectral data from the original description of the compound can be used for dereplication, and so on. The following article describes the dereplication of mycotoxins. Jennessen et al. (J. Agric. Food Chem. 2005, 53, 1833-1840)

Data describing UV/VIS spectra and high resolution mass spectrometry data for the mycotoxin, citrinin, as well as many *Monascus* pigments are described in the Example 5; entitled 'Analysis of LC-DAD-MS Data'.

The screening process employed allowed the identification of *Penicillium* strains that produce *Monascus-like* pigments, as well as the absence of mycotoxin production by the cultured strain. The screening process should, in particular, screen for the mycotoxin citrinin, since it belongs to the same biogenic family as some of the *Monascus* pigments, such as monascin and rubropunctatin.

### I.2 Mycotoxins produced by Penicillium species:

Citrinin is found in almost all *Monascus* pigments. However, *Penicillium* species are known to produce other mycotoxins, *i.e*. cyclopenins, patulin, terrestric acid, 2-methyl isoborneol, ochratoxin A, citrinin, penicillic acid, verrucosidin, penitrem A, asteltoxin, botryodiploidin, chaetoglobosins, communesins, cyclopiazonic acid, verruculogen, isochromantoxins, viriditoxin, mycophenolic acid, PR-toxin, Secalonic acid, rubratoxin, territrems, viridic acid, xanthomegnin, viomellein, vioxanthin, rubratoxin, rugulosin, luteoskyrin, luteospurin, erythroskyrin, emodin, islanditoxin, duclauxin, rugulovasin A and B, rugulosin, spiculisporic acid, cyclochlorotine, and botryodiploidin.

Many *Penicillium* species that produce pigments, produce mycotoxins as well. For instance *P. verrucosum,* which is widespread in cereals in cold climates, produces pigment but also produces two potent toxins viz. ochratoxin A and citrinin. Similarly, *P. persicinum* produces copious amounts of an unknown peach-red pigment in addition to grisefulvin (an antibiotic), and among others the mycotoxins chrysogine and roquefortine C. Further *P. rubrum,* a red pigment producer was found to produce rubratoxin.

### I.3 Pathogenic strains:

Although *Penicillium marneffei* was found to produce large amounts of *Monascus*-like pigments, *P*. *marneffei* is a dangerous pathogen as reported by Liyan et al. (Fifteen cases of penicilliosis in Guangdong, China (2000) Mycopathologia, 158, 151-155) and would therefore not be suitable for use *e.g.* food use. However, several *Monascus*-like pigment-producing strains of *Penicillium* that have not been reported as invasive or pathogenic in humans and did not produce any known mycotoxins, were identified by the described screening process.

### 1.4 Light stability of pigments produced by the selected Penicillium strains:

Pigment compositions of the *Monascus-like* pigments produced by the selected pigment-producing *Penicillium* strains may be tested for their light stability, for example in a beverage-based food system having pH 7. Stability may for example be demonstrated by comparison to that of commercially available *Monascus* pigments, for example using the procedure in Example 5.

### 2. Penicillium strains for Monascus-like pigment production

The following strains can produce *Monascus*-like polyketide based azaphilone pigments without co-production of mycotoxins. Further they are not known to be pathogenic:
*P. neopurpurogenum* (IBT 11181, CBS 238.95, CBS 123796)
*P. ¹neopurpurogenum* (IMI 90178, IBT 4428, IBT 3645, CBS 113154)
*P. ¹neopurpurogenum* (NRRL 1136, IBT 3458, CBS 113153)
*P. ¹neopurpurogenum* (CBS 364.48, IBT 4529)
*P. ¹neopurpurogenum* (NRRL 1748, IBT 3933)
*P. ¹neopurpurogenum* (FRR 75, IBT 4454)
*P. aculeatum* (FRR 2129, IBT 14259, IBT 4185)
*P. ²pseudoaculeatum* (IMI 133243, IBT 14129)
*P. ²rubroaculeatum* (FRR 2005, IBT 14256)
*P. ²rubroaculeatum* (FRR 1664, IBT 14254)
*P. pinophilum* (IMI 114993, IBT 3757)
*P. ³quasipinophilum* (ATCC 9644, IBT 13104)
*P. ⁴neominioluteum* (CCRC 32646, IBT 18368)
*P. punicae* (RMF 81.01, IBT 23082)
*P. synnemafuniculosum* (NRRL 2119, IBT 3954)
*P. amestolkiae* (IMI 147406, IBT 21723)
*P. ⁵monascum* (WSF 3955, IBT 14065, CBS 123797)
¹ Presently classified as *P*. *purpurogenum;* ² Presently classified as *P*. *aculeatum; ³* Presently classified as *P. pinophilum.;* ⁴ Presently classified as *P. minioluteum.; ⁵* new nomenclature should be given.

Beside the discovery of the non-pathogenic and non-toxigenic strains above, some of these strains could be made to produce a *Monascus*-like polyketide based azaphilone pigment that was surprisingly more light-stable than *Monascus* pigment obtained from *Monascus Ruber* (IBT 7904) grown under the same conditions, and even more light-stable than a commercial product: *Monascus* Red (Riken Vitamin Co. Ltd., Japan).

The strains used have been deposited with several different culture collections, as described above. They can be obtained from one or more of the following culture collections:

The IBT Culture Collection of Fungi, Mycology Group, BioCentrum-DTU, Technical University of Denmark; Centraalbureau voor Schimmelcultures (CBS); CABI Bioscience (IMI); Agricultural Research Service Culture Collection (NRRL); FRR culture collection (FRR); American Type Culture Collection (ATCC); Culture Collection and Research Centre, Food Industry Research & Development Institute, Hsinchu, Taiwan (CCRC); Rocky Mountain Fungi collection (RMF); Wisconsin Soil Fungi collection (WSF).

The selected *Penicillium* strains producing *Monascus*-like pigments, can be suitable for industrial application *e.g*. the food or non-food industry, since they may be 'generally accepted as safe' - GRAS strains.

### 3. Method of producing Monascus-like pigment

The general methology employed to grow fungi in order to produce *Monascus*-like pigment is exemplified in the following:
a) providing a liquid-phase growth medium and a solid support,
b) producing a cultivation composition comprising combining the growth medium and the solid support, wherein at least part of the solid support is submerged in the growth medium,
c) inoculating the solid support or the cultivation composition with an inoculum of *Penicillium* spores;
d) cultivating the inoculated cultivation composition of (c);
e) separating the liquid-phase comprising the *Monascus*-like pigment composition.from the product of (d),
f) collecting the liquid-phase.
The one or more *Monascus*-like pigment in the liquid phase can be further isolated by purification and/or concentration steps, such as filtration through a size selective semi-permeable membrane. Optionally, the one or more *Monascus*-like pigment can be extracted from biomass produced in step (d).

### 3.1 Inoculum of spores of a Penicillium strain:

The inoculum preferably comprises 1.2x10 ⁵ to 3x10 ⁵ spores per ml of liquid medium to be inoculated. The spores are derived from a *Penicillium* strain capable of producing one or more *Monascus*-like pigment, that is both non-pathogenic and does not produce any toxin (including mycotoxin), as exemplified above in section (2).

### 3.2 Growth/cultivation conditions for Monascus-like pigment production:

*Penicillium* strains, in general can be cultivated in many ways, such as by submerged cultivation, solid-state fermentation. Using available cultivation technology, *Penicillium* strains provide higher yields of pigment (*Monascus-*like azaphililone pigments) than plant-based colorants (pigments). Furthermore, *Penicillium* strains are shown to secrete copious amounts of extracellular pigments, which facilitates their subsequent extraction and/or isolation, as compared to *Monascus* spp., where pigments accumulate within the *Monascus* biomass.

A submerged cultivation of the *Penicillium* strain inoculum and pigment production would normally comprise the following steps: a) Cultivation in a suitable minimal well-defined medium or complex medium comprising a liquid phase; b) separating the liquid and solid phases of step (a) by centrifugation or filtration to remove biomass from the liquid phase, if the pigment is exogenous; c) optionally, separating the pigment from the liquid phase by acid precipitation followed by centrifugation to separate the precipitate; d) dissolving the pigment in a suitable solvent, such as ethanol, and evaporation of the solvent to obtain a coloured powder. Additionally, the biomass may be extracted using an aqueous alcohol, such as ethanol, to recover pigment that is endogenous or trapped in the biomass.

A solid-state fermentation may use of a variety of starch-based substrates, such as rice and/or leca nuts as possible substrates. The rice could for example subsequently be ground down to yield a coloured powder. The solid substrate may include an agar-based solid phase.

In solid-state fermentation or submerged cultivation, the inoculated growth medium is usually incubated for 7-10 days at 25-30 °C in the dark. However, time of incubation and temperature can be varied.

### 3.4 Growth media for Monascus-like pigment production

Exemplary types of growth media are Yeast extract sucrose (YES) agar; Malt extract agar (MEA), Potato dextrose (PD) agar and Czapek-Dox yeast autolysate (CYA) agar (Frisvad, J. C.; Thrane, U. Mycological media for food-and indoor fungi. In Introduction to Food- and Airborne Fungi. 6th ed.; Samson, R. A., Hoekstra, E. S., Frisvad, J. C., Filtenborg, O., Eds.; Centraalbureau voor Schimmelcultures: Utrecht, The Netherlands, 2002; p 378). Liquid mediums such as Czapek-Dox (CZ) broth would be suitable to use as well. The media type can be changed to provide one or more advantages in the culturing of the *Penicillium* strains, such as for example higher yield or different colour hues. For example, the growth medium may be supplemented with one or more amino acids, such as D- (Ala, Cys, Asp, Glu, Phe, Gly, His, Ile, Lys, Leu, Met, Asn, Pro, Gln, Arg, Ser, Thr, Val, Trp, Tyr), to produce amino acid derivatized *Monascus*-like polyketide based azaphilone pigments with different hues, and increased light stability.

### 3.5 Harvesting and purification of the Monascus-like pigment composition

Depending on the cultivation method, the pigments can be either harvested as the resultant biomass by a conventional procedure such as scraping off of the pigment rich mycelium part from the medium, or recovered by grinding and/or extracting the biomass and the pigmented substrate.

The Penicillium pigments may be recovered from the solid growth media by aqueous extraction combined with filtration, including ultrafiltration, to remove the mycelia and solid media, and the pigment may then be used directly as the aqueous extract or lyophilized for stabilization and storage.

### 4. Parameters for optimising Monascus-like pigment production

Pigment production by *Monascus-like* pigment producing *Penicillium* strains has been analysed under various submerged cultivation conditions in a variety of media, and the results have identified those conditions which promote pigment production over increase in biomass, and can alter the hue of the pigment in the region of orange to red.

### 4.1 Culture conditions

Use of submerged cultivation conditions are preferred for *Penicillium* strains that secrete *Monascus*-like pigments into the surrounding medium. This facilitates the direct recovery of the secreted pigment from the liquid growth medium, avoiding the need to extract pigment from the fungal biomass.

Use of a solid support, in combination with submerged culture conditions is preferred for the production of pigments by *Penicillium* strains. *Penicillium* spores can be inoculated into a submerged culture (comprising a solid support) or pre-inoculated onto a solid support, which is then lowered into the culture medium. The solid support is seen to provide a surface for growth of *Penicillium* biomass, while promoting secondary metabolism resulting in the production of pigments. Since the *Penicillium* biomass produced during cultivation is primarily attached to the solid support, this greatly facilitates the separation of biomass from secreted pigments released into the fermentation growth at the end of cultivation. A variety of solid supports can be used, for example Light Expanded Clay Aggregates (LECA), wood chips, pimp stone cat litter absorbent, Light weight aggregates of fly ash.

Production of pigments by submerged cultivation of *Penicillium* strains on a solid support is further improved by retaining the solid support within a limited portion of the culture medium. This can for example be achieved by introducing the solid support into a semi-permeable container suitable for lowering into the growth medium used for pigment production (Example 7). The container is constructed from a material whose porosity is sufficient to allow free movement of both nutrients in the growth medium and pigments secreted by the *Penicillium* biomass. A suitable material is one that acts as semi-permeable membrane, in the sense that the material has a pore size sufficient to retain biomass and broken cells, which may for example be manufactured from a material selected from paper, plastic, cotton, silk, wool or cellulose or metal wire. The membrane may be constructed from woven or felted fibres or metal wire of the above materials. The membrane may for example be dialysis membrane with a pore size sufficient for free movement of both nutrients in the growth medium and pigments. The container may take the form of a bag with one open end for the introduction of the solid support.
After inoculation of *Penicillium* spores into the solid support, the *Penicillium* biomass that fails to remain associated with the solid support will be retained within the semi-permeable container, while the secreted pigments will diffuse out of the container into the surrounding growth medium. When the fermentation medium comprising the secreted pigment is removed at the end of cultivation, both the major part of the accumulated *Penicillium* biomass as well as cell debri is retained within the semi-permeable container. The subsequent recovery and concentration of the secreted pigments from the harvested fermentation medium by filtration is facilitated by the reduced biomass contamination for example by reducing membrane blockage.

Pigment production during submerged cultivation is enhanced by stirring or shaking the culture (Example 7). Circulation of the growth medium can also be achieved using air pressure.

### 4.2 Growth media tailor-made to enhance pigment production over biomass

A basai growth medium comprising minerai salts and yeast extract (e.g. basal medium in example 8) will support pigment production by *Monascus-*like pigment producing *Penicillium* strains provided that the growth medium is supplemented with low concentrations of a carbon and a nitrogen source. A suitable source of carbon includes lactose or starch e.g. potato starch; while a suitable source of nitrogen includes ammonium nitrate, corn sirup liquor, soybean meal yeast extract.
Preferred concentrations of carbon and nitrogen sources for maximum pigment production are growth medium N1 in example 7, and medium N8 and N9 in example 8.

### 4.3 A process for pigment production

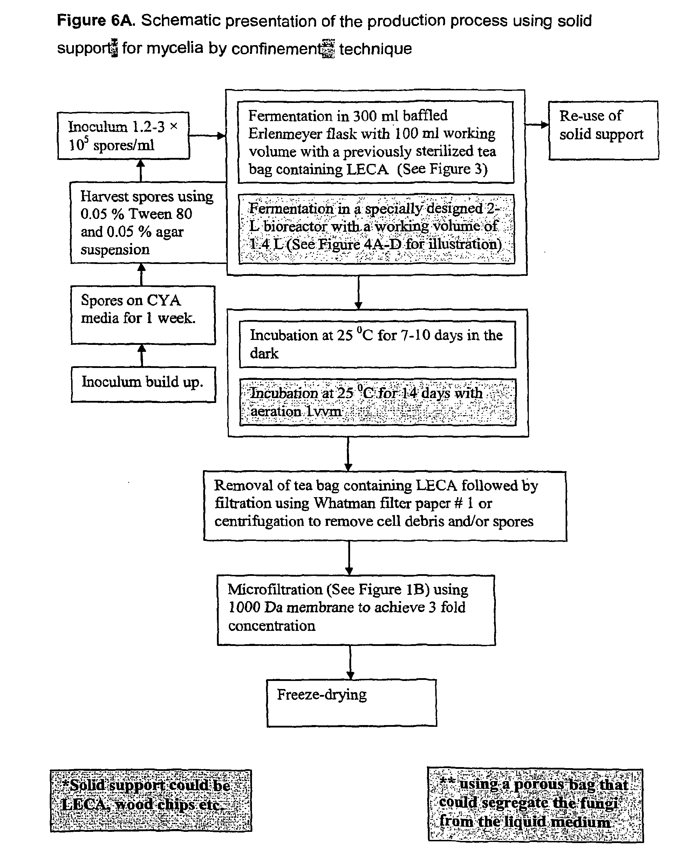

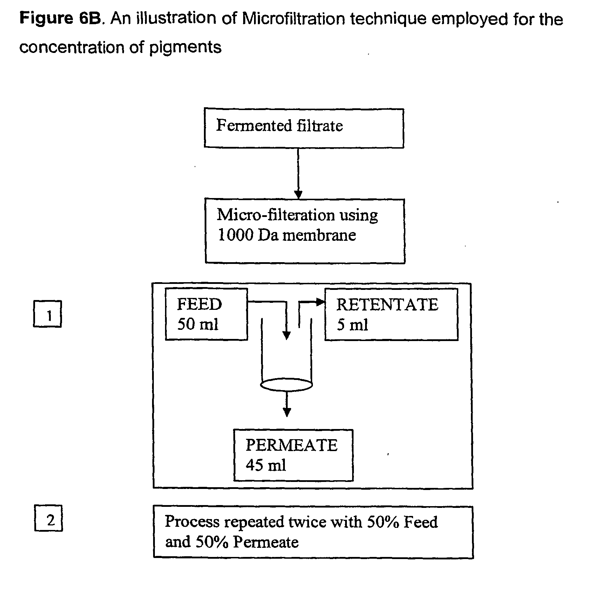

The process steps for fermentative pigment production by a Penicillium strain include (e.g, Figure 6 A,B): submerged cultivated on a solid support; separation of the fermentation broth comprising the secreted pigment from the biomass retained within the semi-permeable container (e.g. tea bag filter); recovery of the pigment from the fermentation broth by filtration {e.g. microfiltration) and optionally concentrating and/or drying (e.g. freeze-drying).

### 5. A bioreactor adapted for pigment production

A specially adapted bioreactor is provided for the commercial scale production of pigments by submerged cultivation of *Penicillium* strains on a solid support, where one embodiment is illustrated in example 11. The features of the bioreactor include the basic essential features of a bioreactor employed for the growth of a microorganism, which include the requirement for a container capable of sterilisation, including at least one opening that can be closed in a manner sufficient to prevent microbial contamination and further provides for inlet and outlet ports for the introduction or exit of air, liquids (e.g. nutrients) or biological samples (e.g. inoculum of spores) or the removal of liquids (e.g. fermentation broth and/or pigmented liquid) or solids (e.g. biomass and/or solid support). The bioreactor may further be provided with a means for circulating the growth medium within the reactor. Such means may include shaking, stir bar, rotatable blade, air pressure supplied via one of the inlets for release of compressed air below the surface of the growth medium.

The bioreactor may further be provided with a means for controlling the temperature within the bioreactor. Suitable means for temperature control include a jacket, or water bath enclosing the bioreactor that is capable of acting as a heat exchanger to either raise or lower the temperature within the bioreactor.

For the purpose of production of pigments, in a solid support is introduced into the bioreactor. The solid support is preferably supported on a metal cage, for example a cage of coiled tubing. In one embodiment, the solid support is retained within a semi-permeable container as detailed under section 4.1. In a preferred embodiment the compressed air is released into the growth medium in close proximity to the solid support, preferably within the semi-permeable container, wherein the solid support is retained.

### 6. Pigment composition and methods for determination

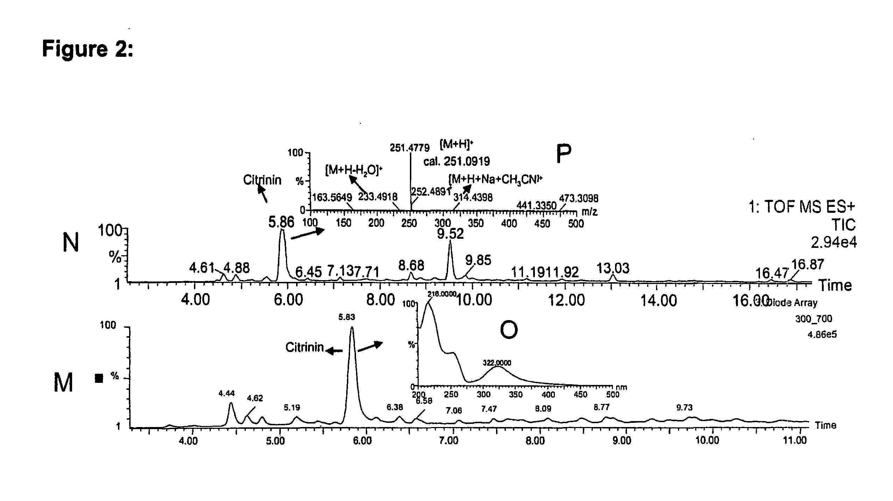

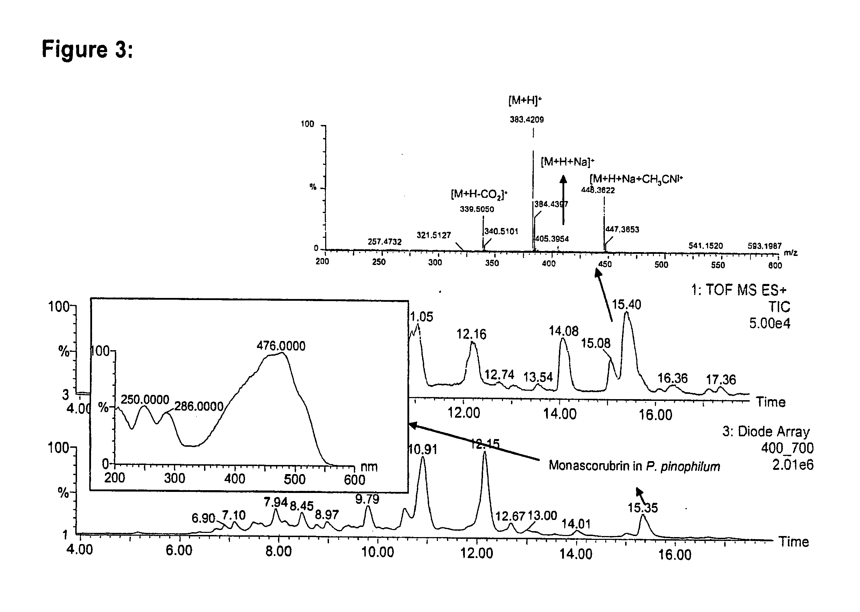

The pigment composition can, for example, be determined by a HPLC chromatogram, with UVNIS DIODE ARRAY and MS detection (see *e.g.* figure 2 and 3). Pigment compositions comprise yellow-orange-red pigments that absorb visible light in the range between 390-530 nm. They are characterised by the absence of known mycotoxins, as well as the presence of *Monascus*-like pigments and/or their derivatives.

*Monascus* and *Monascus*-like pigments are polyketide-based azaphilone pigments, such as: monascorubrin, monascin, monascorubramine, rubropunctatin, rubropunctamine, monascusone A, monascusone B, ankaflavin, xanthomonasin A, mitorubrinol, PP-V, or amino acid derivatives thereof.

The *Monascus-like* polyketide based azaphilone pigments can be derivatives of the above mentioned pigments, such as *e.g.* an amino acid derivative, or a higher or lower homologue, *e.g.* hexyl side chain instead of octyl side chain, wherein the azaphilone molecular framework responsible for the colour in the derivative is unaltered.

This absence of mycotoxins and presence of exemplary *Monascus-like* pigments can be verified by the method of dereplication as previously described.

Some of the pigments are further characterised as being more light-stable than the *Monascus* pigment obtained from *Monascus Ruber* (IBT 7904) grown under the same conditions, and even more light-stable than a commercial product: *Monascus* Red (Riken Vitamin Co. Ltd., Japan). This can be measured as described.

### 7. Use of pigments

The pigments can be used for almost every application of a coloured pigment, *e.g.* food use, non-food use, cosmetic compositions.

The pigment of the present invention can be used as a food colorant, *e.g.* baked goods, baking mixes, beverages and beverage bases, breakfast cereals, cheeses, condiments and relishes, confections and frostings, fats and oils, frozen dairy desserts and mixes, gelatins, puddings and fillings, gravies and sauces, milk products, plant protein products, processed fruits and fruit juices, and snack foods.

The pigment of the present invention can further be used as a non-food colorant, *e.g*. dyeing of textiles, cotton, wool, silk, as well as paints, coatings, inks, and colouring agent for tablets (*e.g*. pharmaceutical tablets), plastic and polymers.

The pigment of the present invention can also be used in cosmetics, *e.g.* in the form of a free, poured or compacted powder, a fluid anhydrous greasy product, an oil for the body and/or the face, a lotion for the body and/or the face, or a hair product. Exemplary uses are such as a make-up, hair-dye or tanning lotion.

### 8. Use of strains to generate colour

The strain itself can be used as a pigment producer within the product to be coloured, as exemplified below.

For certain types of cheeses, it is a common practice to inoculate milk curds with fungal strains to ferment the growth of specific species of mold that gives the cheese a colour, as well as imparting a unique flavour and texture to the cheese. Stilton or Roquefort is created using *Penicillium roqueforti* strains, which are usually non-toxic and are thus safe for human consumption. However, mycotoxins (*e.g*., aflatoxins, roquefortine C, patulin, or others) may accumulate due to fungal spoilage during cheese ripening or storage. *Monascus*-like pigment producing *Penicillium* strains can be used as described above to colour cheese, without the risk of producing mycotoxins, and the strain itself being non-pathogenic.

Beside cheeses, other milk products, baked goods, baking mixes, beverages and beverage bases can be used with the strains described, wherein the strain is providing a colour to the pigment due to cultivation.

### EXAMPLES

### Penicillium strains

The *Penicillium* strains used are:
*P. neopurpurogenum* (IBT 11181, CBS 238.95, CBS 123796)
*P. ¹neopurpurogenum* (IMI 90178, IBT 4428, IBT 3645, CBS 113154)
*P. ¹neopurpurogenum* (NRRL 1136, IBT 3458, CBS 113153)
*P. ¹neopurpurogenum* (CBS 364.48, IBT 4529)
*P. ¹neopurpurogenum* (NRRL 1748, IBT 3933)
*P. ¹neopurpurogenum* (FRR 75, IBT 4454)
*P. aculeatum* (FRR 2129, IBT 14259, IBT 4185)
*P. ²pseudoaculeatum* (IMI 133243, IBT 14129)
*P. ²rubroaculeatum* (FRR 2005, IBT 14256)
*P. ²rubroaculeatum* (FRR 1664, IBT 14254)
*P. pinophilum* (IMI 114993, IBT 3757)
*P. ³quasipinophilum* (ATCC 9644, IBT 13104)
*P. ⁴neominioluteum* (CCRC 32646, IBT 18368)
*P. punicae* (RMF 81.01, IBT 23082)
*P. synnemafuniculosum* (NRRL 2119, IBT 3954)
*P. amestolkiae* (IMI 147406, IBT 21723)
*P. ⁵monascum* (WSF 3955, IBT 14065, CBS 123797)
¹ Presently classified as *P. purpurogenum;* ² Presently classified as *P. aculeatum;* ³ presently classified as *P. pinophilum.;* ⁴ Presently classified as *P. minioluteum.;* ⁵ new nomenclature should be given.

The strains used have been deposited with several different culture collections, as evident from the accession numbers above. They can be obtained from one or more of the following culture collections:

The IBT Culture Collection of Fungi, Mycology Group, BioCentrum-DTU, Technical University of Denmark; Centraalbureau voor Schimmelcultures (CBS); CABI Bioscience (IMI); Agricultural Research Service Culture Collection (NRRL); FRR culture collection (FRR); American Type Culture Collection (ATCC); Culture Collection and Research Centre, Food Industry Research & Development Institute, Hsinchu, Taiwan (CCRC); Rocky Mountain Fungi collection (RMF); Wisconsin Soil Fungi collection (WSF).

### General Production

For preparing an inoculum the strains are revived from the silica gel storage or other storage forms like frozen plugs, etc., by plating onto CYA. After incubation for 7 days at 25 °C in the dark plates are checked for purity. From pure cultures conidial suspension are prepared by transferring fungal conidia into a 14 ml screw cap vial containing 0.1 % agar. The conidial suspension should have a high turbidity before inoculation by a needle onto the production media.

Each of the strains were inoculated in the form of three point inoculations on to YES (Yeast extract 2%; Sucrose 15%; agar 2%) and CYA (NaNO₃ 0.3%; K₂HPO₄ 0.1%; KCI 0.05%; MgSO₄˙7H₂O 0.05%; FeSO₄˙7H₂O 0.001%; Yeast extract 0.5%; Sucrose 3%; agar 2%) media. The plates were incubated in dark at 25 °C for 7 days.

Each of the strains can be cultivated for pigment production on carbohydrate or starch rich solid basic media for 7-10 days at 25-30 °C. From this the pigments can be either harvested as the resultant biomass by a conventional procedure such as scraping off of the pigment rich mycelium part from the medium or recover by grinding the biomass and the pigmented substrate and then lyophilize for stabilization and storage.

The Penicillium pigments may be recovered from the solid growth media by aqueous extraction combined with filtration, including ultrafiltration, to remove the mycelia and solid media, and the pigment may then be used directly as the aqueous extract or lyophilized for stabilization and storage.

### Example 1. Selection of fungi, media, and cultivation conditions

All fungal isolates used in this study were procured from the IBT Culture Collection at BioCentrum-DTU, Technical University of Denmark, Kgs. Lyngby, Denmark. The fungal isolates were listed by the IBT numbers. All fungi were cultivated on either of the four different solid media viz.; Yeast extract sucrose (YES) agar; Malt extract agar (MEA), Potato dextrose (PD) agar and Czapek-Dox yeast autolysate (CYA) agar (Frisvad, J. C.; Thrane, U. Mycological media for food- and indoor fungi. In Introduction to Food- and Airborne Fungi. 6th ed.; Samson, R. A., Hoekstra, E. S., Frisvad, J. C., Filtenborg, O., Eds.; Centraalbureau voor Schimmelcultures: Utrecht, The Netherlands, 2002; p 378) or in specific combinations on which maximum pigment was found to be produced with interesting colour hues in the red to yellow spectra. The cultures were incubated in the dark at 25 °C for 7 days.

The pigment producing fungi that exogenously produced intense colouration on solid media were also grown in liquid medium, Czapek-Dox (CZ) broth with an initial pH adjusted to 6.5. The cultures were incubated in the dark at 25 °C for 7 days. Liquid cultivations were performed in 500 ml baffled Erlenmeyer flasks containing 100 ml of the medium (CZ) at 150 rpm on a rotary shaker at 25 °C for 7 days.

### Example 2. Extraction of fungal pigments

Extraction was carried out by a modified version of the micro-extraction method by Smedsgaard (J. Chromatogr. A 1997, 760, 264-270), where 6 mm plugs were extracted in two steps in a 2 ml vial for 30 minutes, first using 1 ml ethyl acetate with 0.5 % formic acid to break open the cell wall and extract relatively apolar metabolites. The extract so obtained was then transferred to a new 2 ml vial and evaporated *in vacuo.* The second extraction was performed using 1 ml methanol or isopropanol based on our preliminary results indicating maximum pigment extraction from the specific pigment producing fungus. Since the exact chemical nature of pigments varied from fungus to fungus, it was necessary to use the appropriate solvent for the specific strain. By doing so we could extract maximum colour. However, same solvent system was used to extract for the same strains cultured in different media. The second extract was then added to the vial with the residue from the previous extraction. It was then evaporated in a rotational vacuum concentrator (RVC; Christ Martin, Osterode, Germany). Residue was redissolved in 400 µl methanol, in an ultrasonic bath (Branson 2510, Kell-Strom, Wethersfield, USA) for 10 minutes, and filtered through a 0.45 µm PTFE syringe filter (SRI, Eatontown, NJ, USA). This extract was used for chromatographic analysis. In case of the liquid medium CZ, where the pigment was mostly diffused in the media, the colour extract was obtained as per the method used by Jung et al. (J. Agric. Food Chem. 2003, 51, 1302 - 1306).

A schematic presentation of the overall methodology used in the present study is shown in Figure 1. Specific steps and analytical techniques used in the methodology were as follows:

### Example 3. Analysis of pigments

*3.1 Colorimetry:* The absorbance values of pigments in the filtered fermentation broth were adjusted at their respective absorption maxima with purified water, obtained from a Milli-Q system (Millipore, Bedford, MA), as a diluent, so as to measure absorbance within the linearity of Beer-Lambert's law. The dilution factor was then taken into consideration in order to calculate the yield in terms of volumetric production absorbance unit (AU)/100 ml of the broth. The absorption maxima were determined by scanning the extracts for their absorption spectra over the range of 350-700 nm, using a spectrophotometer (Agilent HP 8453, Agilent technologies, Palo Alto, USA).

Absorbance values were also used to determine the color quality. Absorbances recorded at the pigment absorption maxima were two characteristic absorption peaks in the visible region of spectra, the first one at around 495 nm and the other one ranged from 407-420 nm. The ratios of the two absorbances were used to determine the red color index of the pigments, i.e. a higher value of the ratio represents a higher proportion of the red pigments.

### 3.2 Evaluation of pigment composition from the fermentation broth

*A. Solid phase extraction:* The filtrate was subject to clean-up by mixed mode reversed phase-weak anion exchange using Strata-X-AW cartridges (60mg 1ml, Phenomenex, Torrence, CA, USA). Cartridges were conditioned with 1.2 ml methanol and equilibrated with 1.2 ml of water. (MilliQ water, 18.2 µ). 1.2 ml sample acidified with 2% phosphoric acid (in 1:6 proportion) was loaded in a vacuum manifold. The cartridges were then sequentially washed with 1.2 ml water and with 1.2 ml methanol. The pigment mixture, bound in the matrix of the cartridges, was eluted with 1.2 ml of 2% NH₄OH in methanol. The pigment extract so obtained was evaporated to dryness using a rotational vacuum concentrator. It was then re-dissolved in 200 µl of methanol and subjected to LC-HRMS analysis.

### Example 4. Chromatographic Analysis

High-resolution LC-DAD-MS was performed on an Agilent HP 1100 liquid chromatograph (LC) system with a photodiode array detector (DAD) and a 50 x 2 mm i.d., 3 µm, Luna C 18 II column (Phenomenex, Torrance, CA). The LC system was coupled to a LCT orthogonal time-of-flight mass spectrometer (Waters-Micromass, Manchester, U.K.) with a Z-spray electrospray ionization (ESI) source, and a LockSpray probe and controlled by the MassLynx 4.0 software. MS system was operated in either the positive or negative ESI mode using a water-acetonitrile gradient system starting from 15% acetonitrile, which was increased linearly to 100% in 20 minutes with a holding time of 5 minutes or starting from 5% acetonitrile for 2 minutes and increasing to 100% in 18 minutes and keeping it for 5 minutes. The water was buffered with 10 mM ammonium formate and 20 mM formic acid and the acetonitrile with 20 mM formic acid. The instrument was tuned to a resolution > 7000 (at half peak height). The method is well established and described by Nielsen et al. (J. Agric. Food Chem. 2005, 53, 8190-8196).
The LC method was further developed by using a 50 mm × 2mm i.d., 3 µm, Luna PFP column held at 25 °C. A sample volume of 3 µl was injected and eluted at a flow rate of 0.2ml / min using water with 0.1% formic acid and acetonitrile with 0.1 % formic acid gradient system. The gradient started from 10% acetonitrile and increased linearly to 60 % in 12 minutes, and further to 80 % in 10 minutes and finally to 100 % in another 5 minutes with a holding time of 5 minutes. This was used in combination with solid phase extraction to analyse filtered fermentation pigment extracts.

### Example 5. Analysis of LC-DAD-MS Data

The presence of metabolites involved in the present study was detected in ESI+ or ESI- from the first scan function of the reconstructed ion chromatograms and the UV/VIS spectrum was obtained after background subtraction. The DAD-MS data for each of these compounds are shown below.

Ankaflavin. Ankaflavin was detected ESI+ as m/z 387.2004 [M + H]+ and confirmed by the adducts m/z 409.1803 [M + H + Na]+ and 450.2213 [M + H + Na + CH3CN]+. The UV-vis spectrum was λmax: 232, 283 (shorter peak) and 390.

Monascin. Monascin was detected in ESI+ as m/z 359.1719 [M + H]+ and confirmed by the adducts m/z 397.1389 [M + H + K]+ and 422.1926 [M + H + Na + CH3CN]+. The UV-vis spectrum was λmax: 230, 288 (shorter peak) and 394.

Rubropunctatin. Rubropunctatin was detected in ESI+ as m/z 355.4232 [M + H]+ and confirmed by the adduct m/z 418.3666 [M + H + Na + CH3CN]+, and the fragment m/z 311.5018 [M + H - CO2]+. The UV-vis spectrum was λmax: 246, 288, and 474.

Rubropunctamine. Rubropunctamine was detected in ESI+ as m/z 354.4623 [M + H]+ and confirmed by the adducts 376.4212 [M + H + Na]+ and 417.3899 [M + H + Na + CH3CN]+ and the fragment 310.5256 [M + H - CO2]+. The UV-vis spectrum was λmax: 306, 414, and 524 with a shoulder at 252.

N-glutarylrubropunctamine. N-glutarylrubropunctamine was detected as m/z 484.20 [M + H]⁺ and confirmed by the adducts 506.21 [M + H + Na]+ and 547.22 [M + H + Na + CH3CN]+. The UV-vis spectrum was λmax: 250, 274, 430, and 522.

Citrinin. Standard citrinin was detected in ESI+ as m/z 251.4779 [M + H]+ and confirmed by the adduct 314.4398 [M + H + Na + CH3CN]+ and the fragment 233.4918 [M + H - H2O]+. The UV spectrum was λmax: 216, 322 with a shoulder at 242.

Monascorubrin. Monascorubrin was detected in ESI+ as m/z 383.4141 [M + H]+ and confirmed by the adducts 405.3971 [M + H + Na]+ and 446.3614 [M + H + Na + CH3CN]+ and the fragment 339.5067 [M + H - CO2]+ The UV-vis spectrum was λmax: 248, 288, and 478.

Xanthomonasin A. Xanthomonasin A was detected in ESI+ as m/z 389.4427 [M + H]+ and confirmed by the adducts 411.4379 [M + H + Na]+ and 452.3948 [M + H + Na + CH3CN]+ and the fragment 361.4615 [M + H - H20]+. The UV-vis spectrum was λmax: 236, 288 (shorter), and 392.

PP-V. PP-V (homologue of monascorubramine, 3-(9a-methyl-3-octanoyl-2,9-dioxo-2,7,9,9a-tetrahydro-furo[3,2-g]isoquinolin-6-yl) acrylic acid) was detected in ESI+ as m/z 412.3929 [M + H]+ and confirmed by the adducts 434.3493 [M + H + Na]+ and 475.3170 [M + H + Na + CH3CN]+ and the fragment 368.4687 [M + H - CO2]+. The UV-vis spectrum was λmax: 302, 420, and 52 with a shoulder at 252.

Threonine derivative of rubropunctatin. Threonine derivative of rubropunctatin was detected in ESI+ as m/z 456.3559 [M + H]+ and confirmed by the adducts 478.3064 [M + H + Na]+ and 519.2740 [M + H + Na + CH3CN]+. UV-vis spectrum was λmax: 204, 282, 424, and 524.

N-glutarylmonascorubramine. N-glutarylmonascorubramine was detected as m/z 512.23 [M + H]⁺ and confirmed by the adduct 534.21 [M + H + Na]+. The UV-vis spectrum was λmax: 212 (with a shoulder at 272), 420, and 504.

Monascorubramine was not detected in the pigment extracts under study.

The presence of PP-R (7-(2-hydroxyethyl)-monascorubramine) was detected in ESI+ from the first scan function of the reconstructed ion chromatograms of m/z 426.4288 [M + H]+ and confirmed by the fragments m/z 448.3817 [M + H + Na]+ and 489.3471 [M + H + Na + CH3CN]+.

### Example 6. Light stability of Monascus-like pigments

Three of the representative fungal pigment compositions were added to a beverage-based food system having pH 7 in canted-neck flasks that were filled to the brim to avoid oxidation. The filled flasks were subjected to a light dose in a light cabinet (Altas suntest XLS, xenon lamp) at 25 °C. CIELAB (see *e.g.* Mapari et al., J. Agric. Food Chem. 2006, 54(19), 7027-7035), and coordinates were measured on a chromameter (Minolta CT 310, Konica Minolta, Mahwah NJ) before the light exposure and over a time period until the test samples were almost decolourised. Prior, the L-value of the samples was adjusted to a specific value based on an optimal value normally used in the food application (For example for red colorants the L-value was adjusted from 75 to 78). From the CIELAB values ΔE value, a value which is an indicator of color loss based on both a* and b* values taken together with the L-value, was calculated. The intensity of light in the light cabinet was 168 J/cm².

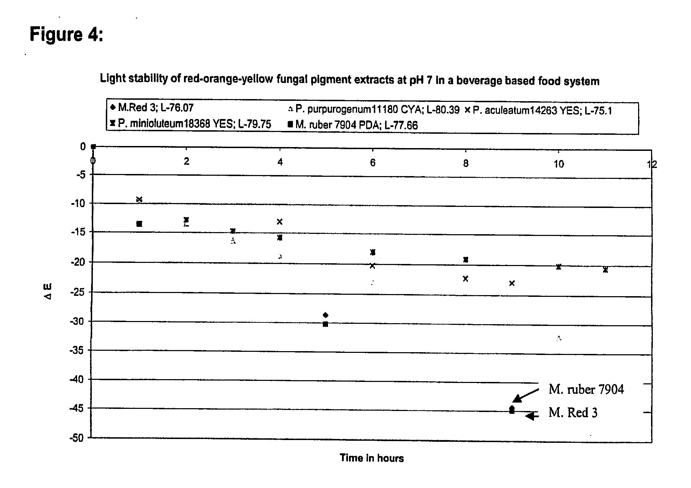

Figure 4 shows the enhanced light-stability of the *Monascus*-like pigments produced by 3 species of Penicillium (P. purpurogenum; P. aculeatum and P. minioluteum) compared to commercially available *Monascus* pigments.

### Example 7. Yeast extract and shaking during submerged cultivation enhance pigment production by Penicillium purpurogenum IBT 4529

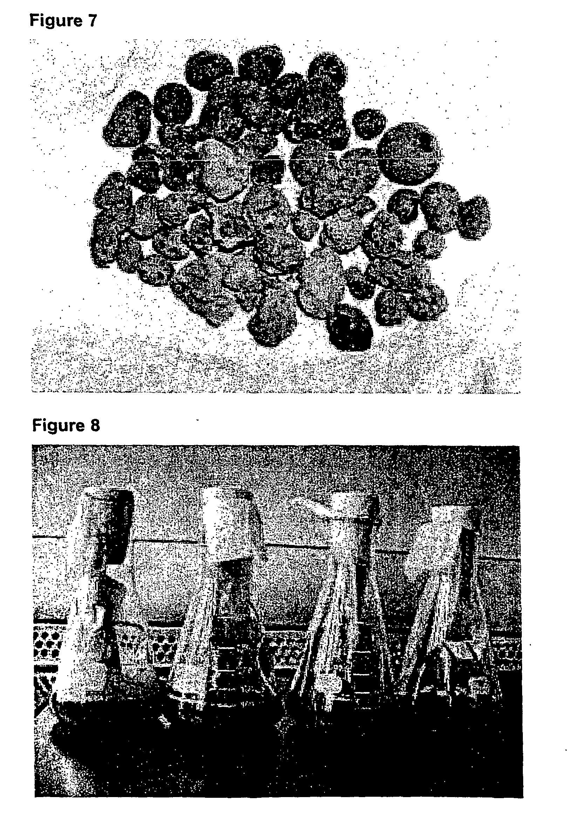

Pigment production by *Penicillium purpurogenum* IBT 4529, cultivated in 300 ml baffled Erlenmeyer culture flasks on a solid support with 100 ml growth medium (Figure 8), was measured in the presence or absence of a yeast extract supplement, according to the method set out schematically in Figure 6A.

Solid support: comprised approximately 8-9 grams of Light Expanded Clay Aggregates [LECA] (Figure 7),retained within a tea filter bag and sterilized by autoclaving.

Growth medium: Czapeck-Dox (CZ) fermentation broth having the following composition was used as the media in the presence or absence 1% yeast extract (YE).

| **Media ingredient:** | **Quantity** |
|---|---|
| Sucrose | 30 g |
| NaNO₃ | 3g |
| K₂HPO₄ | 1 g |
| MgSO₄.7H₂O 0.5 | g |
| FeSO₄. 7H₂O | 0.01 g |
| KCI | 0.5 g |
| Distilled water | 1000 ml |
| pH before autoclaving | 5.5 |

Spores of *P. purpurogenum* IBT 4529 were inoculated directly onto the LECA contained within the tea filter, which was then transferred into the flask comprising the above growth medium. The experiment was performed in duplicate. The cultures were incubated at 25 °C in the dark, either under stationary or shaking conditions (120rpm). After 7 days incubation, the tea filter comprising the majority of the fungal biomass adhered to LECA was removed from the flask, and the remaining fermentation broth was filtered through Whatman filter paper # 1 to remove residual biomass and the absorbance values of the filtrate was recorded at their absorption maxima.

**Table 1**

| Volumetric pigment production (AU/100ml) | | | | |
|---|---|---|---|---|
| Media | Culture conditions | OD₄₉₄ | OD₄₁₈ | OD₄₉₄/ OD₄₁₈ |
| CZ | Stationary | 0.423 | - | |
| CZ | Shaking | 1.184 | 0.871 | 1.36 |
| CZ+YE | Stationary | 0.550 | 0.444 | 1.24 |
| CZ+YE | Shaking | 3.87 | 2.65 | 1.46 |

The results illustrated in Table 1 show that shaking conditions and the presence of 1 % yeast extract strongly favoured *Monascus*-like pigment production by *P. purpurogenum* IBT 4529.

### Example 8. The supply of carbon and nitrogen sources modulate pigment production by Penicillium purpurogenum IBT 11181

Pigment production by *Penicillium purpurogenum* IBT 11181, cultivated at 25 °C in the dark under shaking conditions (120 rpm) in the presence of different amounts and/or ratios of carbon and nitrogen sources according to the protocol defined in Example 7.
Inoculum: 1.0 × 10⁵ spores/ml *Penicillium purpurogenum* IBT 11181
Solid support: tea filter with LECA: total wt: 3.25 ± 0.19 g
Growth medium: composition of basal medium is as follows:

| **Media ingredient:** | **Quantity** |
|---|---|
| Yeast extract | 5 g |
| K₂HPO₄ | 1 g |
| MgSO₄.7H₂O | 0.5 g |
| FeSO₄. 7H₂O | 0.01 g |
| KCl | 0.5 g |
| Distilled water | 1000 ml |
| pH before autoclaving | 5.5 |

The basal medium was supplemented with a carbon source, comprising Potato starch (PS) at a concentration range of 5-100 g/L and lactose (L) at a concentration range of 10-150 g/L; and a nitrogen source, comprising corn steep liquor (CSL) and ammonium nitrate (AN) each at a concentration range of 3-30 g/L, which was supplied in the combination, determined using the Umetrics software MODDE7 for screening purposes using fractional factorial design, and set out Table 2.

After 7 days cultivation, the fermentation broth was treated as set out in Example 7, and the absorbance of the filtrate was recorded as shown below.

**Table 2**

| **Expt./Media** | **Concentration (g/L)** | | | | **Volumetric production (AU/100ml)** | | | **Biomass (g)** |
|---|---|---|---|---|---|---|---|---|
| | **PS** | **L** | **CSL** | **AN** | **OD₄₉₅** | **OD₄₀₇** | **OD₄₉₅/ OD₄₀₇** | |
| N1 | 3 | 3 | 5 | 10 | 1.89 | 1.28 | 1.48 | 0.97 |
| N2 | 3 | 30 | 5 | 150 | 1.15 | 0.98 | 1.17 | 2.1 |
| Control | - | - | - | - | OD₄₈₃ | 0.288 | - | 1.22 |

| | | | | | | | | |
|---|---|---|---|---|---|---|---|---|
| (CZ media plus 0.5% yeast extract) | | | | | | | | |

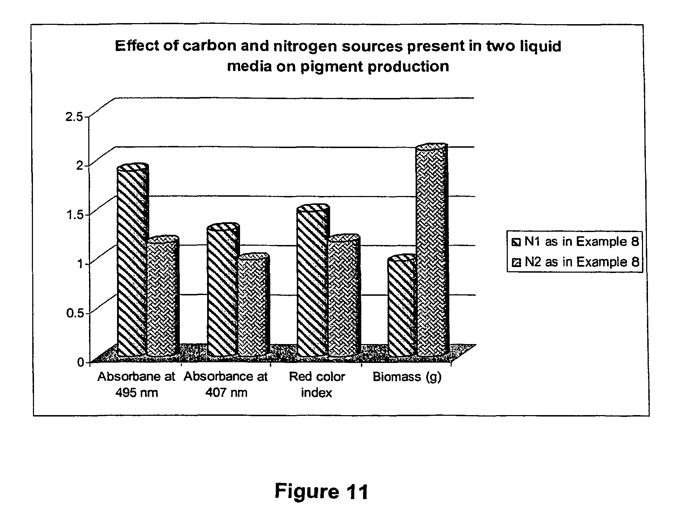

The results in Table 2 (Figure 11) show that manipulation of the concentration of carbon and/or nitrogen sources results in a quantitative increase or decrease in the red color hue (OD₄₉₅/ OD₄₀₇ values are positively correlated with increase in red hue). A carbon to nitrogen ratio in the medium corresponding to N1 favors pigment production over biomass, while a lower concentration range of carbon and nitrogen sources (as in N1) promotes red pigment production. Cultivation in the other growth medium compositions (not shown), where the concentration of carbon and/or nitrogen sources was higher than N1, either resulted in less pigment (as in N2) or no pigment.

### Example 9. Media formulation for tailor-made production of red-orange pigments by Penicillium purpurogenum IBT 11181 strain.

Pigment production by *Penicillium purpurogenum* IBT 11181, cultivated at 25 °C in the dark under shaking conditions (120 rpm) for 10 days was performed according to the protocol defined in Example 7, and further detailed below.
Inoculum: 3 x 10⁵ spores/ml *Penicillium purpurogenum* IBT 11181
Solid support: tea filter with LECA
Growth medium: composition of basal medium as defined in example 8. The basal medium was supplemented with a carbon source, comprising Potato starch (PS) at a concentration range of 0.5-2.75 g/L and lactose (L) at a concentration range of 0.1- 3 g/L; and a nitrogen source, comprising corn steep liquor (CSL) and ammonium nitrate (AN) each at a concentration range of 3-30 g/L, which was supplied in a combination determined using reduced Central Composite Face-centred (CCF) based quadratic design (Unmetrics).

**Table 3**

| **Expt/ Media** | **Concentration (g/L)** | | | | **Volumetric production (AU/100ml)** | | |
|---|---|---|---|---|---|---|---|
| | **AN** | **PS** | **L** | **CSL** | **OD₄₉₀** | **OD₄₂₀** | **OD₄₉₀/OD₄₂₀** |
| N3 | 0.1 | 0.5 | 1 | 0.1 | 0.282 | 0.281 | 1.00 |
| N4 | 0.1 | 5 | 1 | 0.1 | 0.233 | 0.071 | 3.28 |
| N5 | 3 | 0.5 | 10 | 0.1 | 0.138 | 0.075 | 1.84 |
| N6 | 0.1 | 5 | 10 | 0.1 | 0.429 | 0.071 | 6.04 |
| N7 | 3 | 0.5 | 10 | 3 | 1.325 | 0.285 | 4.65 |
| N8 | 0.1 | 2.75 | 5.5 | 1.55 | 0.735 | 0.215 | 3.42 |
| N9 | 1.55 | 2.75 | 1 | 1.55 | 1.36 | 0.83 | 1.64 |
| N10 | 1.55 | 2.75 | 5.5 | 0.1 | **1.703** | **1.09** | 1.57 |
| Center Point Replicate | | | | | | | |
| N11 | 1.55 | 2.75 | 5.5 | 1.55 | ***1.81 ± 0.03*** | ***1.08 ± 0.01*** | 1.68 |
| Control | - | - | - | - | 0.941 | 0.927 | 1.02 |

| | | | | | | | |
|---|---|---|---|---|---|---|---|
| (CZ media plus 0.5% yeast extract) | | | | | | | |

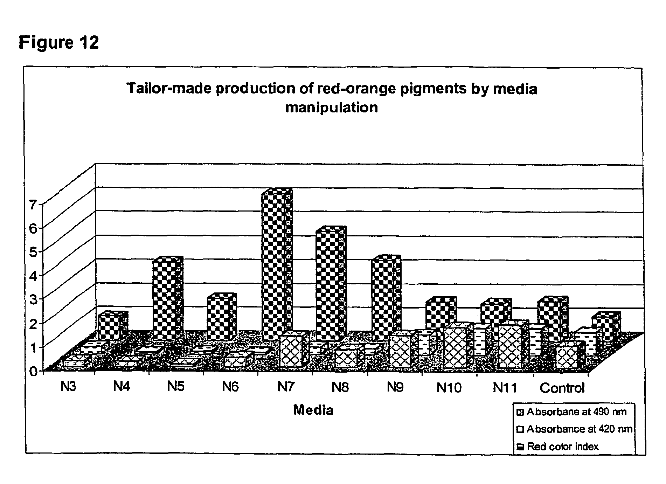

The results in Table 3 (Figure 12) show that maximum pigment production was obtained on medium N11, closely followed by medium N10. The color hue of the pigment produced was almost the same in the N10 and N11 media, since they had similar OD₄₉₀/ OD₄₂₀ ratios. It is also possible to produce pigments with different hues of red-orange by changing the combination of carbon and/or nitrogen sources, as it can be seen from the increasing or decreasing ratio of OD₄₉₀/ OD₄₂₀ (N6, N7, and N8 in Table 3). The red pigment production was increased in N4 medium as compared to N3 medium when potato starch concentration increased from 0.5 g/L to 5g/L keeping the other ingredients same. Comparing N5 and N7, an increase in the concentration of CSL from 0.1 to 3 g/L, in the medium N7, while keeping the other ingredients unchanged, resulted in both a higher pigment yield and gave a pigment with relatively more red color hue. This implies manipulation of carbon and/or nitrogen sources can be used to produce tailor-made colorants.

### Example 10. N11 growth medium is also tailor-made for pigment production in Penicillium purpurogenum strain IBT 3645.

Pigment production by *Penicillium purpurogenum* IBT 3645, cultivated at 25 °C in the dark under shaking conditions (120 rpm) for 10 days was performed with two biological replicates according to the protocol defined in Example 9, and further detailed below.
Inoculum: 3 × 10⁵ spores/ml *Penicillium purpurogenum* IBT 3645
(corresponding to IMI 90178, IBT 4428, and CBS 113154)
Growth medium: N11 comprising basal medium supplemented with C/N sources as given in Example 11.

**Table 4**

| | **Volumetric production (AU/100ml)** | | |
|---|---|---|---|
| **Broth Type** | **OD₄₉₆** | **OD₄₁₆** | **OD₄₉₆ OD₄₁₆** |
| Filtered Fermentation broth | 3.77 ± 0.41 | 2.64 ± 0.27 | 1.43 |

Table 4 clearly indicates that *P*. *purpurogenum* strain IBT 3645 produced a 2-2.4 fold higher yield of pigment (as measured by absorbance at the two absorption maxima) in the N11 media.

### Example 11. A bioreactor adapted for production of pigments by Monascus-like pigment producing Penicillium strains

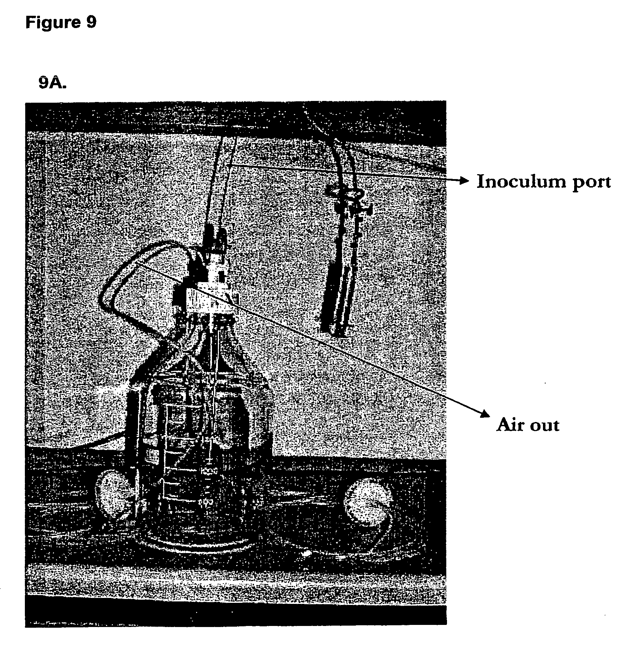

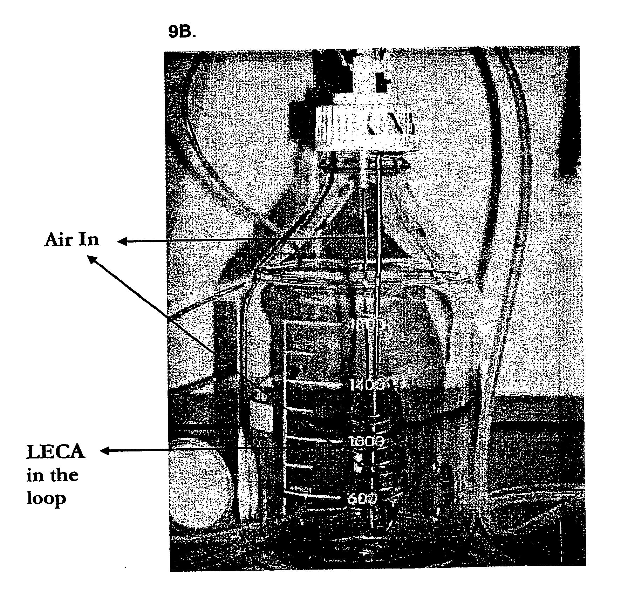

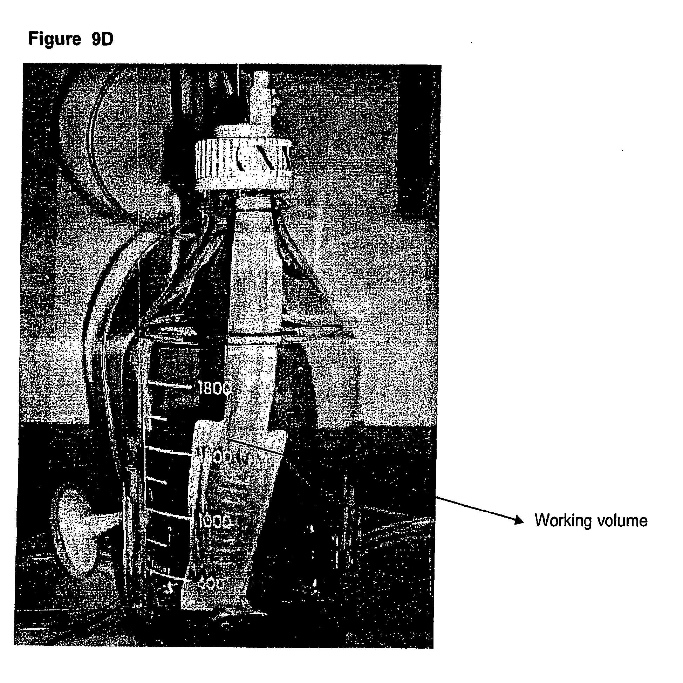

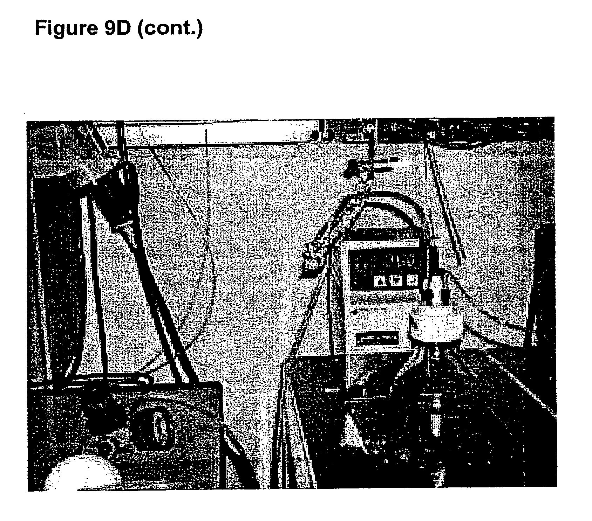

The bioreactor, shown in Figure 9A-C, is specifically adapted for scaled-up production of pigments from *Penicillium* strains and is suitable for use in the production process presented in Figure 6A and B.
The bioreactor is a closable container designed to accommodate a solid support, such as LECA, and is further supplied with a mixing devise. In the present examples, the container is a 2-L capped bottle with a working volume of 1.4 L (Figure 9A and B), or a 1-L capped bottle with a working volume of 0.6 L (Figure 9C and D), further provided with a means for mixing. Mixing is provided by an aerating device comprising a tube located within the container, wherein one end of the tube is connected to an air-inlet capable of supplying sterile air under pressure. The container is further provided with an air-outlet, connected to the other end of the tube, allowing air to exit from the container. The tube may include a region formed as a coiled loop. In the present example, the solid support (LECA) placed within the container, is retained within the coiled loop of the aerating devise. The LECA solid support within the loop, is further retained within a tea filter bag, which is placed over the coiled loop of the aerating devise.

The 1-L bioreactor was tested for pigment production. The growth medium and components of the bioreactor were sterilized separately. The media was added to the bioreactor under the aseptic conditions in a laminar flow hood. Inoculation was carried out using the inoculation port. Fermentation conditions were as follows:
Inoculum: 3 × 10⁵ spores/ml *Penicillium purpurogenum* IBT 11181
Solid support: LECA retained within coil and tea filter
Growth medium: N1 media from Example 8 (0.6L volume)

The bioreactor container was incubated under stationary conditions in a water bath, maintained at 25 °C, as shown in Figure 5 D, for 14 days. Sterile air (1vvm) was provided to achieve mixing and transfer of growth media nutrients in and out of the tea filter.

After fermentation, the tea filter and solid support were removed from the container, and the fermentation broth was decanted and filtered through Whatman filter paper # 1. Pigments adhered to the biomass retained on the filter paper and/or LECA was recovered by washing with 70% ethanol on a shaker at 120 rpm overnight at room temperature. The absorbance values of the filtered fermentation broth as well as the ethanolic extract were recorded.

**Table 3**

| | **Volumetric production (AU/100ml)** | | |
|---|---|---|---|
| **Broth Type** | **OD₄₉₅** | **OD₄₁₅** | **OD₄₉₅/ OD₄₁₅** |
| Filtered Fermentation Broth | 22.8 ± 0.04 | 14.4 ± 0.05 | 1.58 |

| | **OD₅₀₉** | **OD₄₁₅** | **OD₅₀₉/ OD₄₁₅** |
|---|---|---|---|
| Ethanolic Extract | 5.72 ± 0.008 | 4.86 ± 0.008 | 1.18 |

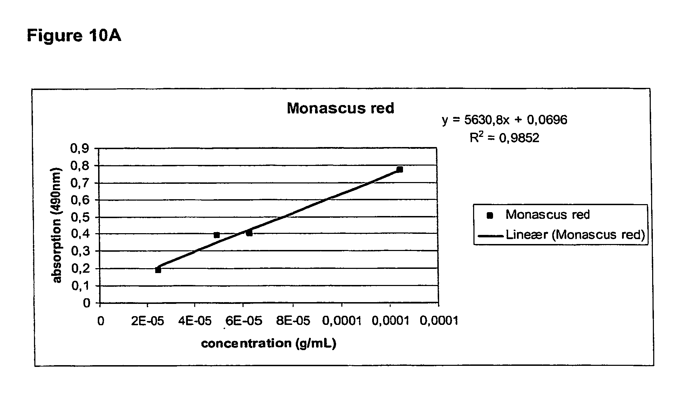

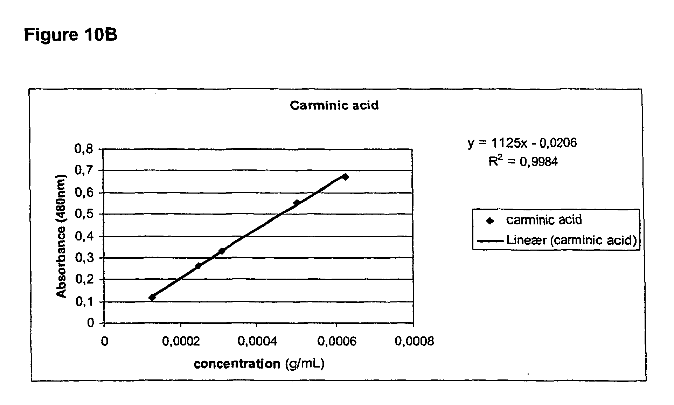

Volumetric production of pigments present in the filtered fermentation broth was compared to the commercially available Monascus Red colorant (Riken Vitamin Co. Ltd., Japan and Carminic acid (Chr. Hansen A/S, Denmark) as standards (see Figure 10A and 10B). The extrapolation of absorbance values at 490nm for Monascus Red and 480 nm for Carminic acid showed the production titre to be **0.6g/L** in terms of Monascus Red equivalent and **3.16 g/L** in terms of Carminic acid equivalent. Note that, methods of pigment production from *Penicillium* strains according to the present invention, are a considerable economic improvement over known methods which require as many as 14000 (carmine-producing) insects to produce 100 g of the natural food colorant, carmine (Mapari et al, Curr. Opin. Biotechnol., 16:231-238)

### Example 12. Identification of individual pigments in Monascus-like pigment compostions produced by Penicillium strains.

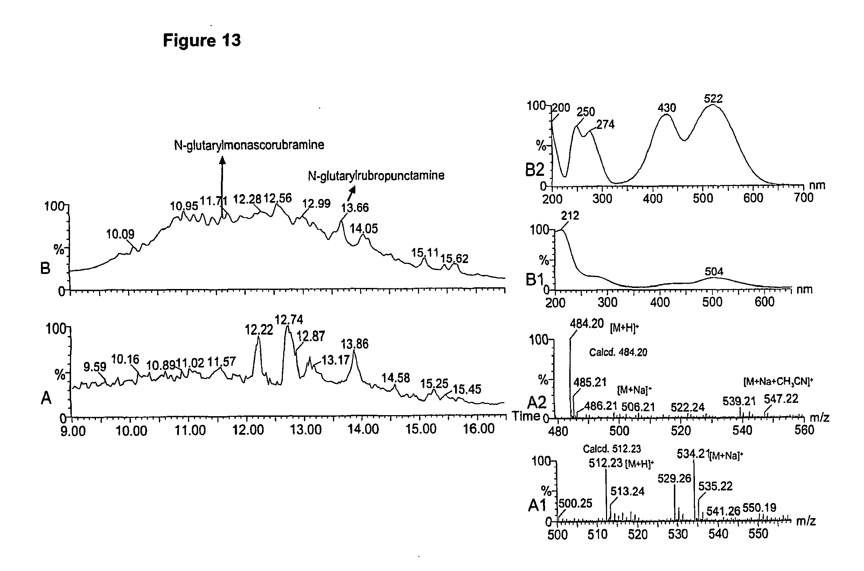

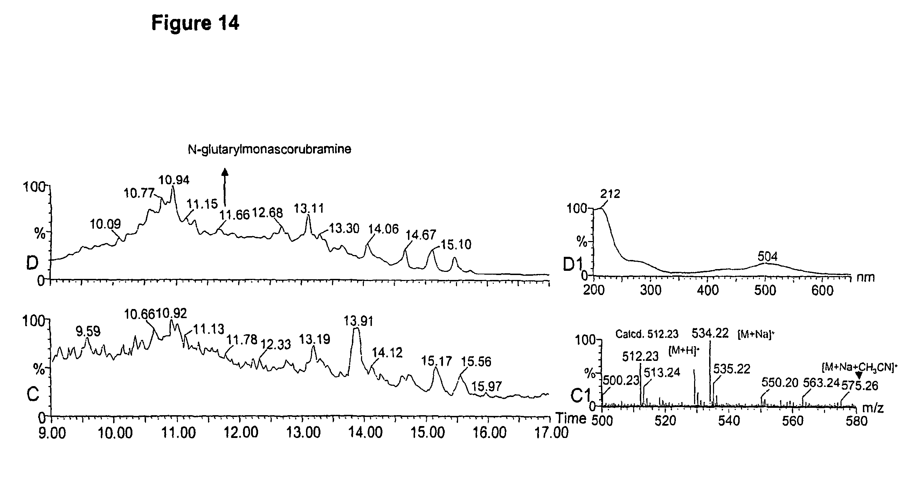

*Monascus-like* pigments, produced by *Penicillium purpurogenum* IBT 11181 in control medium as in Example 9, according to the method of the present invention, are identified as the water-soluble pigments: N-glutarylmonascorubramine (B1), and N-glutarylrubropunctamine (Figure 13 (B2)). Similarly, *Monascus-like* pigments, produced by *Penicillium purpurogenum* IBT 3645 in N11 medium as in Example 10, according to the method of the present invention, are identified as the water-soluble pigment: N-glutarylmonascorubramine (Figure 14 (D1)).

These pigments, present in the partially purified pigment extract of the fermentation medium, were detected and identified by means of total ion chromatogram (A), and UV-vis chromatogram at 400-700 nm (B), high-resolution mass spectrum (A1) and UV-vis spectrum.

### Example 13. Method of colouring of cheese

A cheese cultivation medium is inoculated with one or more of the mentioned *penicillium* strains, which is allowed to grow at 25 °C with agitation and aeration of the mixture continuously for 7 days. This mixture should be further processed into a cheese. Following a ripening the cheese is harvested and will be processed to produce a coloured cheese.

## Claims

1. Method for producing a *Monascus*-like pigment composition comprising:
a) providing a liquid-phase growth medium and a solid support,
b) producing a cultivation composition comprising combining the growth medium and the solid support, wherein at least part of the solid support is submerged in the growth medium, and wherein the solid support is retained within a semi-permeable container,
c) inoculating the solid support or the cultivation composition with an inoculum of *Penicillium* spores;
d) cultivating the inoculated cultivation composition of (c);
e) separating the liquid-phase comprising the *Monascus*-like pigment composition.from the product of (d).

2. The method of claim 1, wherein the solid support is selected from among Light Expanded Clay Aggregates (LECA), wood chips, cat litter, fly ash aggregates (LWA), and pimp stone.

3. The method of claims 1 or 2, wherein the semi-permeable container is manufactured from a material selected from among paper, silk, wool, plastic, metal wire and cellulose.

4. The method of any one of claims 1-3, wherein the liquid-phase growth medium is circulated during cultivation.

5. The method of any one of claims 1-4, wherein the inoculum is inoculated onto the solid support.

6. The method of any one of claims 1-5, wherein the *Penicillium* spores are derived from at least one of the following strains: *P. neopurpurogenum* (IBT 11181, CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *P. neopurpurogenum* (FRR 75, IBT 4454), *P. aculeatum* (FRR 2129, IBT 14259, IBT 4185), *P*. *pseudoaculeatum* (IMI 133243, IBT 14129), *P. rubroaculeatum* (FRR 2005, IBT 14256), *P. rubroaculeatum* (FRR 1664, IBT 14254), *P. pinophilum* (IMI 114993, IBT 3757), *P. quasipinophilum* (ATCC 9644, IBT 13104), *P. neominioluteum* (CCRC 32646, IBT 18368), *P. punicae* (RMF 81.01, IBT 23082), *P. synnemafuniculosum* (NRRL 2119, IBT 3954), *P. amestolkiae* (IMI 147406, IBT 21723), *P. monascum* (WSF 3955, IBT 14065, CBS 123797).

7. The method of any one of claims 1-5, wherein the pigment is monascorubrin and the *Penicillium* spores are derived from *Penicillium pinophilum* IBT 13104.

8. A *Monascus*-like pigment composition prepared according to the method of claim 6 or 7.

9. Method for producing a *Monascus*-like pigment composition, comprising:
a) providing an inoculum;
b) inoculating a growth medium with the inoculum;
c) cultivating the inoculated growth medium of b);
d) harvesting the biomass product of c); and
e) isolating the *Monascus-like* pigment composition from the biomass or the growth medium,
wherein the inoculum is selected from among at least one of the following strains: *P. neopurpurogenum* (IBT 11181, CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *P. neopurpurogenum* (FRR 75, IBT 4454), *P. aculeatum* (FRR 2129, IBT 14259, IBT 4185), *P. pseudoaculeatum* (IMI 133243, IBT 14129), *P. rubroaculeatum* (FRR 2005, IBT 14256), *P. rubroaculeatum* (FRR 1664, IBT 14254), *P. pinophilum* (IMI 114993, IBT 3757), *P. ³quasipinophilum* (ATCC 9644, IBT 13104), *P. neominioluteum* (CCRC 32646, IBT 18368), *P. punicae* (RMF 81.01, IBT 23082), *P. synnemafuniculosum* (NRRL 2119, IBT 3954), *P. amestolkiae* (IMI 147406, IBT 21723), *P. monascum* (WSF 3955, IBT 14065, CBS 123797).

10. Method according to claim 9, wherein the growth medium is supplemented with one or more amino acids.

11. A *Monascus-like* pigment composition prepared according to the method of claim 9 or 10.

12. Method for colouring a food product and/or non-food product employing a *Monascus-like* pigment composition comprising:
a) providing an inoculum;
b) inoculating a growth medium with the inoculum;
c) cultivating the inoculated growth medium of b) including the food product and/or non-food product; and
d) harvesting the coloured food product and/or non-food product of c); wherein the inoculum is selected from among at least one of the following strains: *P. neopurpurogenum* (IBT 11181, CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *P*. *neopurpurogenum* (FRR 75, IBT 4454), *P*. *aculeatum* (FRR 2129, IBT 14259, IBT 4185), *P. pseudoaculeatum* (IMI 133243, IBT 14129), *P*. *rubroaculeatum* (FRR 2005, IBT 14256), *P*. *rubroaculeatum* (FRR 1664, IBT 14254), *P*. *pinophilum* (IMI 114993, IBT 3757), *P. ³quasipinophilum* (ATCC 9644, IBT 13104), *P.neominioluteum* (CCRC 32646, IBT 18368), *P.punicae* (RMF 81.01, IBT 23082), *P*. *synnemafuniculosum* (NRRL 2119, IBT 3954), P. *amestolkiae* (IMI 147406, IBT 21723), *P*. *monascum* (WSF 3955, IBT 14065, CBS 123797).

13. Method according to claim 12, wherein the food product is one of the following: baked goods, baking mixes, beverages and beverage bases, cheeses, and milk products.

14. Use of the *Monascus*-like pigment composition according to claim 8 or 11, as a colouring agent for a food product and/or non-food product.

15. Use according to claim 14, wherein the food product is one of the following: baked good, baking mix, beverage and beverage base, breakfast cereal, cheese, condiment and relish, confection and frosting, fat and oil, frozen dairy dessert and mix, gelatin, pudding and filling, gravy and sauce, milk product, plant protein product, processed fruit and fruit juice, and snack food.

16. Use according to claim 15, wherein the non-food product is one of the following: textile, cotton, wool, silk, leather, paper, paint, polymer, plastic, inks, tablet.

17. Cosmetic composition comprising the *Monascus*-like pigment composition according to claim 8 or 11.

18. Cosmetic composition according to claim 17, in the form of a free, poured or compacted powder, a fluid anhydrous greasy product, an oil for the body and/or the face, a lotion for the body and/or the face, or a hair product.

19. Cosmetic composition according to claims 17 or 18, which is a make-up composition.

20. A bioreactor adapted for the production of a *Monascus-like* pigment composition by penicillium strains, comprising a closable container for receiving a liquid growth medium, wherein the container is provided with a means for mixing; an inlet and outlet port for aeration of the container; an inlet port and outlet port for introduction and removal of biological material; **characterised in that** the bioreactor is provided with a LECA solid support retained within the container, and wherein said solid support is further retained within a semi-permeable container, said solid support being capable of at least partial submersion in the liquid growth medium when present into the container.

21. The bioreactor of claim 20, wherein the semi-permeable container is manufactured from a semi-permeable material selected from among paper, silk, wool, plastic and cellulose.

## Patentansprüche

1. Verfahren zur Herstellung von einer *Monascus*-ähnlichen Pigmentzusammensetzung, umfassend:
a) Bereitstellen von einem Flüssigphasenwachstumsmedium und einer festen Auflage,
b) Herstellen von einer Kultivierungszusammensetzung umfassend das Kombinieren von dem Wachstumsmedium und der festen Auflage, wobei mindestens ein Teil der festen Auflage in das Wachstumsmedium eingetaucht wird, und wobei die feste Auflage in einem halbdurchlässigen Behälter aufbewahrt wird,
c) Inokulieren der festen Auflage oder der Kultivierungszusammensetzung mit einem Inokulum von *Penicillium*-sporen-,
d) Kultivieren der inokulierten Kultivierungszusammensetzung von (c);
e) Trennen der Flüssigphase mit der *Monascus*-ähnlichen Pigmentzusammensetzung von dem Produkt von (d).

2. Verfahren nach Anspruch 1, wobei die feste Auflage unter Light Expanded Clay Aggregates (LECA), Hackschnitzel, Katzenstreu, Flugascheaggregate (LWA) und Bimsstein ausgewählt ist.

3. Verfahren nach Anspruch 1 oder 2, wobei der halbdurchlässige Behälter aus einem Material hergestellt ist, das unter Papier, Silke, Wolle, Kunststoff, Metalldraht und Zellulose ausgewählt ist.

4. Verfahren nach irgendeinem der Ansprüche 1-3, wobei das Flüssigphasenwachstumsmedium während des Kultivierens umgewälzt wird.

5. Verfahren nach irgendeinem der Ansprüche 1-4, wobei das Inokulum auf der festen Auflage inokuliert wird.

6. Verfahren nach irgendeinem der Ansprüche 1-5, wobei die *Penicillium-*sporen von mindestens einem der folgenden Stämme abgeleitet sind: *P*. *neopurpurogenum* (IBT 11181 , CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *P. neopurpurogenum* (FRR 75, IBT 4454), *P. aculeatum* (FRR 2129, IBT 14259, IBT 4185), *P*. *pseudoaculeatum* (IMI 133243, IBT 14129), P. *rubroaculeatum* (FRR 2005, IBT 14256), *P. rubroaculeatum* (FRR 1664, IBT 14254), *P. pinophilum* (IMI 114993, IBT 3757), *P. quasipinophilum* (ATCC 9644, IBT 13104), *P. neominioluteum* (CCRC 32646, IBT 18368), *P. punicae* (RMF 81.01 , IBT 23082), *P. synnemafuniculosum* (NRRL 2119, IBT 3954), *P. amestolkiae* (IMI 147406, IBT 21723), *P. monascum* (WSF 3955, IBT 14065, CBS 123797).

7. Verfahren nach irgendeinem der Ansprüche 1-5, wobei das Pigment Monascorubrin ist, und die *Penicillium-sporen von Penicillium pinophilum* IBT 13104 abgeleitet sind.

8. *Monascus*-ähnliche Pigmentzusammensetzung, die nach dem Verfahren nach Anspruch 6 oder 7 hergestellt ist.

9. Verfahren zur Herstellung von einer *Monascus*-ähnlichen Pigmentzusammensetzung, umfassend:
a) Bereitstellen eines Inokulums;
b) Inokulieren eines Wachstumsmedium mit dem Inokulum;
c) Kultivieren des inokulierten Wachstumsmediums von b);
d) Ernten des Biomassenprodukts von c); und
e) Abtrennen der *Monascus*-ähnlichen Pigmentzusammensetzung von der Biomasse oder dem Wachstumsmedium,
wobei das Inokulum unter mindestens einem der folgenden Stämme ausgewählt ist: *P. neopurpurogenum* (IBT 11181 , CBS 238.95, CBS 123796), *P*. *neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *P*. *neopurpurogenum* (FRR 75, IBT 4454), *P. aculeatum* (FRR 2129, IBT 14259, IBT 4185), *P. pseudoaculeatum,* (IMI 133243, IBT 14129), *P. rubroaculeatum* (FRR 2005, IBT 14256), *P. rubroaculeatum* (FRR 1664, IBT 14254), *P. pinophilum* (IMI 114993, IBT 3757), *P*. *³quasipinophilum* (ATCC 9644, IBT 13104), *P. neominioluteum* (CCRC 32646, IBT 18368), *P. punicae* (RMF 81.01 , IBT 23082), *P. synnemafuniculosum* (NRRL 2119, IBT 3954), *P. amestolkiae* (IMI 147406, IBT 21723), *P. monascum* (WSF 3955, IBT 14065, CBS 123797).

10. Verfahren nach Anspruch 9, wobei das Wachstumsmedium mit einer oder mehreren Aminosäuren ergänzt wird.

11. *Monascus*-ähnliche Pigmentzusammensetzung, die nach dem Verfahren nach Anspruch 9 oder 10 hergestellt ist.

12. Verfahren zum Einfärben eines Nahrungsmittels und/oder eines Non-Food-Produkts durch Anwendung einer *Monascus*-ähnlichen Pigmentzusammensetzung, umfassend:
a) Bereitstellen eines Inokulums;
b) Inokulieren eines Wachstumsmediums mit dem Inokulum;
c) Kultivieren des inokulierten Wachstumsmediums von b) einschließlich des Nahrungsmittels und/oder des Non-Food-Produkts, und
d) Ernten des eingefärbten Nahrungsmittels und/oder Non-Food-Produkts von c); wobei das Inokulum unter mindestens einem der folgenden Stämme ausgewählt ist: *P. neopurpurogenum* (IBT 11181 , CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P*. *neopurpurogenum* (CBS 364.48, IBT 4529), *P. neopurpurogenum* (NRRL 1748, IBT 3933), *P. neopurpurogenum* (FRR 75, IBT 4454), *P. aculeatum* (FRR 2129, IBT 14259, IBT 4185), *P. pseudoaculeatum* (IMI 133243, IBT 14129), *P. rubroaculeatum* (FRR 2005, IBT 14256), *P. rubroaculeatum* (FRR 1664, IBT 14254), *P. pinophilum* (IMI 114993, IBT 3757), P. *³quasipinophilum* (ATCC 9644, IBT 13104), *P. neominioluteum* (CCRC 32646, IBT 18368), *P*. *punicae* (RMF 81.01 , IBT 23082), *P. synnemafuniculosum* (NRRL 2119, IBT 3954), *P. amestolkiae* (IMI 147406, IBT 21723), *P. monascum* (WSF 3955, IBT 14065, CBS 123796).

13. Verfahren nach Anspruch 12, wobei das Nahrungsmittel eins von den folgenden ist: Gebäck, Backmischungen, Getränke und Getränkebasen, Käse und Milchprodukte.

14. Anwendung der *Monascus*-ähnlichen Pigmentzusammensetzung nach Anspruch 8 oder 11 als ein Färbemittel für ein Nahrungsmittel und/oder ein Non-Food-Produkt.

15. Anwendung nach Anspruch 14, wobei das Nahrungsmittel eins von den folgenden ist: Gebäck, Backmischungen, Getränke und Getränkebasen, Frühstücksflocken, Käse, Gewürz und Relish, Konfekt und Zuckerguss, Fett und Öl, gefrorene Milchdesserts und -Mischungen, Gelatine, Pudding und Füllung, Soße und Dressing, Milchprodukte, Pflanzenproteinprodukte, verarbeitetes Obst und Obstsaft und Knabberzeug.

16. Anwendung nach Anspruch 15, wobei das Non-Food-Produkt eins von den folgenden ist: Textile, Baumwolle, Wolle, Silke, Leder, Papier, Farbe, Polymer, Kunststoff, Tinte, Schreibtafeln.

17. Kosmetikzusammensetzung umfassend die *Monascus*-ähnliche Pigmentzusammensetzung nach Anspruch 8 oder 11.

18. Kosmetikzusammensetzung nach Anspruch 17 in Form eines freien, gegossenen oder verdichteten Pulvers, eines flüssigen, wasserfreien, fettigen Produkts, eines Körper- und/oder Gesichtsöls, einer Körper- und/oder Gesichtslotion oder eines Haarprodukts.

19. Kosmetikzusammensetzung nach Anspruch 17 oder 18, die eine Make-up-Zusammensetzung ist.

20. Bioreaktor für die Herstellung einer *Monascus*-ähnlichen Pigmentzusammensetzung durch Penicillium-Stämme, umfassend einen schließbaren Behälter zur Aufnahme eines flüssigen Wachstumsmediums, wobei der Behälter mit einem Mittel zum Mischen; einem Einlass- und Auslasskanal zur Lüftung des Behälters; einem Einlass- und Auslasskanal zur Einführung und Entfernung von biologischem Material vorgesehen ist; **dadurch gekennzeichnet, dass** der Bioreaktor mit einer festen LECA-Auflage versehen ist, die im Behälter aufbewahrt wird, und wobei die feste Auflage weiter in einem halbdurchlässigen Behälter aufbewahrt wird, wobei die feste Auflage mindestens teilweise in das flüssige Wachstumsmedium eingetaucht werden kann, wenn im Behälter vorhanden.

21. Bioreaktor nach Anspruch 20, wobei der halbdurchlässige Behälter aus einem halbdurchlässigen Material hergestellt ist, die unter Papier, Silke, Wolle, Kunststoff und Zellulose ausgewählt ist.

## Revendications

1. Procédé de production d'une composition de pigment de *Monascus,* comprenant:
a) la réalisation d'un milieu de croissance en phase liquide et un support solide,
b) la production d'une composition de culture comprenant la combinaison du milieu de croissance et du support solide, dans lequel au moins une partie du support solide est immergée dans le milieu de croissance, et dans lequel le support solide est retenu à l'intérieur d'un conteneur semi-perméable,
c) l'inoculation du support solide ou de la composition de culture avec un inoculum de spores de *Penicillium;*
d) la culture de la composition de culture inoculée de (c);
e) la séparation de la phase liquide comprenant la composition de pigment de *Monascus à* partir du produit de (d).

2. Procédé selon la revendication 1, dans lequel le support solide est sélectionné parmi les agrégats légers expansés d'argile (LECA (Light Expanded Clay Aggregate)), les copeaux de bois, la litière pour chat, les granulats de cendres volantes (GCV), et la pierre ponce.

3. Procédé selon la revendication 1 ou 2, dans dans lequel le conteneur semi-perméable est fabriqué à partir d'un matériau sélectionné parmi le papier, la soie, la laine, le plastique, le fil métallique et la cellulose.

4. Procédé selon l'une quelconque des revendications 1 à 3, dans lequel le milieu de croissance en phase liquide est circulé pendant la culture.

5. Procédé selon l'une quelconque des revendications 1 à 4, dans lequel l'inoculum est inoculé sur le support solide.

6. Procédé selon l'une quelconque des revendications 1 à 5, dans lequel les spores de *Penicillium* sont issues d'au moins une des souches suivantes: *P*. *neopurpurogenum* (IBT 11181, CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *P. neopurpurogenum* (FRR 75, IBT 4454), *P. aculeatum* (FRR 2129, IBT 14259, IBT 4185), *P*. *pseudoaculeatum* (IMI 133243, IBT 14129), *P*. *rubroaculeatum* (FRR 2005, IBT 14256), *P. rubroaculeatum* (FRR 1664, IBT 14254), *P. pinophilum* (IMI 114993, IBT 3757), *P. quasipinophilum* (ATCC 9644, IBT 13104), *P. neominioluteum* (CCRC 32646, IBT 18368), *P. punicae* (RMF 81.01, IBT 23 082), *P. synnemafuniculosum* (NRRL 2119, IBT 3954), *P. amestolkiae* (IMI 147406, IBT 21723), *P. monascum* (WSF 3955, IBT 14065, CBS 123797).

7. Procédé selon l'une quelconque des revendications 1 à 5, dans lequel le pigment est monascorubrin et les spores de *Penicillium* sont dérivées de *Penicillium pinophilum* IBT 13104.

8. Composition de pigment de *Monascus* préparée selon le procédé selon la revendication 6 ou 7.

9. Procédé de production d'une composition de pigment de *Monascus,* comprenant:
a) la réalisation d'un inoculum;
b) l'inoculation d'un milieu de croissance avec l'inoculum;
c) la culture du milieu de croissance inoculé de b) ;
d) la récolte du produit de la biomasse de la c); et
e) l'isolement de la composition de pigment de *Monascus* de la biomasse ou du milieu de croissance,
l'inoculum étant sélectionné parmi au moins l'une des souches suivantes: *P*. *neopurpurogenum* (IBT 11181, CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *P. neopurpurogenum* (FRR 75, IBT 4454), *P. aculeatum* (FRR 2129, IBT 14259, IBT 4185), *p. pseudoaculeatum* (IMI 133243, IBT 14129), *P*. *rubroaculeatum* (FRR 2005, IBT 14256), *P. rubroaculeatum* (FRR 1664, IBT 14254), *P. pinophilum* (IMI 114993, IBT 3757), *P. ³quasipinophilum* (ATCC 9644, IBT 13104), *P. neominioluteum* (CCRC 32646, IBT 18368), *P. punicae* (RMF 81.01, IBT 23 082), *P. synnemafuniculosum* (NRRL 2119, IBT 3954), *P. amestolkiae* (IMI 147406, IBT 21723), *P. monascum* (FSM 3955, IBT 14065, CBS 123797).

10. Procédé selon la revendication 9, dans lequel le milieu de croissance est complété par un ou plusieurs acides aminés.

11. Composition de pigment de *Monascus* préparée selon le procédé selon l'une quelconque des revendications 9 ou 10.

12. Procédé pour la coloration d'un produit alimentaire et/ou d'un produit non alimentaire en utilisant une composition de pigment de *Monascus* comprenant:
a) la réalisation d'un inoculum;
b) l'inoculation d'un milieu de croissance avec l'inoculum;
c) la culture du milieu de croissance inoculé de b) comprenant le produit alimentaire et/ou le produit non alimentaire; et
d) la récolte du produit alimentaire et/ou du produit non alimentaire colorées de c);
où l'inoculum est sélectionné parmi au moins l'une des souches suivantes: *P*. *neopurpurogenum* (IBT 11181, CBS 238.95, CBS 123796), *P. neopurpurogenum* (IMI 90178, IBT 4428, IBT 3645, CBS 113154), *P. neopurpurogenum* (NRRL 1136, IBT 3458, CBS 113153), *P. neopurpurogenum* (CBS 364.48, IBT 4529), *P. neopurpurogenum* (NRRL 1748, IBT 3933), *p. neopurpurogenum* (FRR 75, IBT 4454), *P. aculeatum* (FRR 2129, IBT 14259, IBT 4185), *p. pseudoaculeatum* (IMI 133243, IBT 14129), *P*. *rubroaculeatum* (FRR 2005, IBT 14256), *P. rubroaculeatum* (FRR 1664, IBT 14254), *P. pinophilum* (IMI 114993, IBT 3757), *P. ³quasipinophilum* (ATCC 9644, IBT 13104), *P. neominioluteum* (CCRC 32646, 18368 IBT), *P. punicae* (RMF 81.01, IBT 23082), *P. synnemafuniculosum* (NRRL 2119, IBT 3954), *P. amestolkiae* (IMI 147406, IBT 21723), *P. monascum* (WSF 3955, IBT 14065, CBS 123797).

13. Procédé selon la revendication 12, dans lequel le produit alimentaire est l'un des éléments suivants: les produits de boulangerie, les mélanges à pâtisserie, les boissons et les bases de boissons, les fromages et les produits laitiers.

14. Usage de la composition de pigment de *Monascus* selon la revendication 8 ou 11, en tant qu'un agent de coloration d'un produit alimentaire et/ou d'un produit non alimentaire.

15. Usage selon la revendication 14, dans lequel le produit alimentaire est l'un des suivants: les produits de boulangerie, les mélanges de pâtisserie, les boissons et la base de boissons, les céréales de petit déjeuner, le fromage, les condiments et le relish, la confiserie et le glaçage, la graisse et l'huile, les desserts et mélanges laitiers congelés, la gélatine, le pudding et le remplissage, les sauces et les jus, les produits laitiers, les produits de protéines végétales, les fruits traités et les jus de fruits, et les produits de grignotage.

16. Usage selon la revendication 15, dans lequel le produit non-alimentaire est l'un des suivants: le textile, le coton, la laine, la soie, le cuir, le papier, la peinture, les polymères, le plastique, l'encre, le comprimé.

17. Composition cosmétique comprenant la composition de pigment de *Monascus* selon la revendication 8 ou 11.

18. Composition cosmétique selon la revendication 17, dans la forme d'une poudre libre, coulée ou compactée, un produit gras et anhydre de fluide, une huile pour le corps et/ou le visage, une lotion pour le corps et/ou le visage, ou un produit capillaire.

19. Composition cosmétique selon la revendication 17 ou 18 qui est une composition de maquillage.

20. Bioréacteur adapté pour la production d'une composition de pigment de *Monascus* par des souches de penicillium, comprenant un conteneur à fermer pour recevoir un milieu de croissance liquide, dans lequel le conteneur est fourni d'un moyen de mélange; un orifice d'entrée et de sortie pour l'aération du conteneur; un orifice d'entrée et de sortie pour l'introduction et l'enlèvement du matériel biologique; **caractérisé en ce que** le bioréacteur est pourvu d'un support solide fait de LECA et retenu à l'intérieur du conteneur, et ledit support solide étant en outre retenu à l'intérieur d'un conteneur semi-perméable, ledit support solide étant capable d'au moins une submersion partielle dans le milieu de croissance liquide quand il est présent dans le conteneur.

21. Bioréacteur selon la revendication 20, dans lequel le conteneur semi-perméable est fabriqué à partir d'un matériau semi-perméable sélectionné parmi le papier, la soie, la laine, le plastique et la cellulose.
